# EUROPÄISCHE PATENTSCHRIFT

(11) **EP 1 146 881 B1**
(45) Veröffentlichungstag und Bekanntmachung des Hinweises auf die Patenterteilung: **04.01.2006**
(21) Anmeldenummer: 00909001.0
(22) Anmeldetag: 04.02.2000
(51) Int. Cl.: A61K 31/70, A61K 31/66, A61K 38/21, A61K 39/42, A61K 39/235, A61K 39/245, G01N 33/50, A61P 35/00

(54) **MITTEL ZUR PRÄVENTION UND/ODER BEHANDLUNG EINER GEWEBEVERÄNDERUNG MESENCHYMALEN URSPRUNGS**
PREPARATION FOR THE PREVENTION AND/OR TREATMENT OF A TISSUE CHANGE OF MESENCHYMAL ORIGIN
AGENT POUR PREVENIR ET/OU TRAITER LES MODIFICATIONS TISSULAIRES D'ORIGINE MESENCHYMATEUSE

(30) Priorität: 04.02.1999 DE 19904514; 13.09.1999 DE 19943786; 13.09.1999 DE 19943787
(43) Veröffentlichungstag der Anmeldung: 24.10.2001
(73) Patentinhaber: Bullerdiek, Jörn, 28355 Bremen (DE)
(72) Erfinder: Bullerdiek, Jörn, 28355 Bremen (DE)
(74) Vertreter: Bohmann, Armin K.
(86) Internationale Anmeldenummer: PCT/DE2000/000364
(87) Internationale Veröffentlichungsnummer: WO 2000/045851

(56) Entgegenhaltungen:
- WO-A-96/16629
- WO-A-98/04286
- DATABASE BIOSIS [Online] BIOSCIENCES INFORMATION SERVICE, PHILADELPHIA, PA, US; 1992 BEAN B: "ANTIVIRAL THERAPY CURRENT CONCEPTS AND PRACTICES" Database accession no. PREV199294002244 XP002148093 & CLINICAL MICROBIOLOGY REVIEWS, Bd. 5, Nr. 2, 1992, Seiten 146-182, ISSN: 0893-8512
- DATABASE BIOSIS [Online] BIOSCIENCES INFORMATION SERVICE, PHILADELPHIA, PA, US; 1997 WUTZLER PETER: "Antiviral therapy of herpes simplex and varicella-zoster virus infections." Database accession no. PREV199800388307 XP002148094 & INTERVIROLOGY, Bd. 40, Nr. 5-6, 1997, Seiten 343-356, ISSN: 0300-5526
- DATABASE BIOSIS [Online] BIOSCIENCES INFORMATION SERVICE, PHILADELPHIA, PA, US; 1994 ROBAK TADEUSZ: "Biological properties and clinical applications of interferon gamma (IFN-gamma)." Database accession no. PREV199497282211 XP002148095 & ACTA HAEMATOLOGICA POLONICA, Bd. 25, Nr. 1, 1994, Seiten 19-29, ISSN: 0001-5814
- DATABASE BIOSIS [Online] BIOSCIENCES INFORMATION SERVICE, PHILADELPHIA, PA, US; 1987 BABIUK L A ET AL: "USE OF RECOMBINANT BOVINE ALPHA-1 INTERFERON IN REDUCING RESPIRATORY DISEASE INDUCED BY BOVINE HERPESVIRUS TYPE 1" Database accession no. PREV198784049679 XP002148096 & ANTIMICROBIAL AGENTS AND CHEMOTHERAPY, Bd. 31, Nr. 5, 1987, Seiten 752-757, ISSN: 0066-4804
- DATABASE BIOSIS [Online] BIOSCIENCES INFORMATION SERVICE, PHILADELPHIA, PA, US; 1993 BALK A H M M ET AL: "Passive immunization against cytomegalovirus in allograft recipients: The Rotterdam Heart Transplant Program experience." Database accession no. PREV199396125288 XP002148097 & INFECTION, Bd. 21, Nr. 4, 1993, Seiten 195-200, ISSN: 0300-8126
- DATABASE BIOSIS [Online] BIOSCIENCES INFORMATION SERVICE, PHILADELPHIA, PA, US; 1981 SIEGAL F P ET AL: "FATAL DISSEMINATED ADENOVIRUS 11 PNEUMONIA IN AN AGAMMA GLOBULINEMIC PATIENT" Database accession no. PREV198273083204 XP002148098 & AMERICAN JOURNAL OF MEDICINE, Bd. 71, Nr. 6, 1981, Seiten 1062-1067, ISSN: 0002-9343
- LAUF R ET AL: "PASSIVE IMMUNIZATION OF MARMOSET MONKEYS AGAINST NEOPLASIA INDUCED BY A HERPESVIRUS" NATURE (LONDON), Bd. 255, Nr. 5505, 1975, Seiten 226-228, XP002148092 ISSN: 0028-0836
- DATABASE BIOSIS [Online] BIOSCIENCES INFORMATION SERVICE, PHILADELPHIA, PA, US; 1997 PRING-AKERBLOM P ET AL: "PCR-based detection and typing of human adenoviruses in clinical samples." Database accession no. PREV199799692858 XP002148099 & RESEARCH IN VIROLOGY, Bd. 148, Nr. 3, 1997, Seiten 225-231, ISSN: 0923-2516
- DATABASE BIOSIS [Online] BIOSCIENCES INFORMATION SERVICE, PHILADELPHIA, PA, US; 1997 KUWANO KAZUYOSHI ET AL: "Detection of group C adenovirus DNA in small-cell lung cancer with the nested polymerase chain reaction." Database accession no. PREV199799709133 XP002148100 & JOURNAL OF CANCER RESEARCH AND CLINICAL ONCOLOGY, Bd. 123, Nr. 7, 1997, Seiten 377-382, ISSN: 0171-5216
- DATABASE BIOSIS [Online] BIOSCIENCES INFORMATION SERVICE, PHILADELPHIA, PA, US; 1977 JONSSON N ET AL: "STUDIES ON ADENOVIRUS TYPE 9 INDUCED MAMMARY FIBRO ADENOMAS IN RATS AND THEIR MALIGNANT TRANSFORMATION" Database accession no. PREV197764058172 XP002148101 & CANCER (PHILADELPHIA), Bd. 39, Nr. 6, 1977, Seiten 2513-2519, ISSN: 0008-543X
- KRISHNAN RAMESH ET AL: "Epstein-Barr virus induced renal leiomyoma." JOURNAL OF UROLOGY, Bd. 161, Nr. 1, Januar 1999 (1999-01), Seite 212 XP000946176 ISSN: 0022-5347
- DATABASE BIOSIS [Online] BIOSCIENCES INFORMATION SERVICE, PHILADELPHIA, PA, US; 1996 JAVIER RONALD ET AL: "Mammary tumors induced by human adenovirus type 9: A role for the viral early region 4 gene." Database accession no. PREV199699041789 XP002148102 & BREAST CANCER RESEARCH AND TREATMENT, Bd. 39, Nr. 1, 1996, Seiten 57-67, ISSN: 0167-6806
- DATABASE BIOSIS [Online] BIOSCIENCES INFORMATION SERVICE, PHILADELPHIA, PA, US; 1991 JAVIER R ET AL: "HUMAN ADENOVIRUS TYPE 9-INDUCED RAT MAMMARY TUMORS" Database accession no. PREV199192030807 XP002148103 & JOURNAL OF VIROLOGY, Bd. 65, Nr. 6, 1991, Seiten 3192-3202, ISSN: 0022-538X
- DATABASE BIOSIS [Online] BIOSCIENCES INFORMATION SERVICE, PHILADELPHIA, PA, US; 1997 GALLEGO MARTA I ET AL: "Complex genomic rearrangement within the 12q15 multiple aberration region induced by integrated human papillomavirus 18 in a cervical carcinoma cell line." Database accession no. PREV199799646478 XP002148104 & MOLECULAR CARCINOGENESIS, Bd. 19, Nr. 2, 1997, Seiten 114-121, ISSN: 0899-1987
- DATABASE BIOSIS [Online] BIOSCIENCES INFORMATION SERVICE, PHILADELPHIA, PA, US; 1995 BERLINGIERI MARIA TERESA ET AL: "Inhibition of HMGI-C protein synthesis suppresses retrovirally induced neoplastic transformation of rat thyroid cells." Database accession no. PREV199598189052 XP002148105 & MOLECULAR AND CELLULAR BIOLOGY, Bd. 15, Nr. 3, 1995, Seiten 1545-1553, ISSN: 0270-7306
- DATABASE MEDLINE [Online] US NATIONAL LIBRARY OF MEDICINE (NLM), BETHESDA, MD, US; GEURTS J M ET AL: "Molecular characterization of a complex chromosomal rearrangement in a pleomorphic salivary gland adenoma involving the 3'-UTR of HMGIC." retrieved from STN Database accession no. 97312600 XP002148106 & CANCER GENETICS AND CYTOGENETICS, (1997 JUN) 95 (2) 198-205. ,
- BULLERDIEK JOERN: "Leiomyoma-Do viruses play the main role?" GENES CHROMOSOMES & CANCER, Bd. 26, Nr. 2, Oktober 1999 (1999-10), Seite 181 XP000946177 ISSN: 1045-2257

### SEQUENZPROTOKOLL

<110> Bullerdiek, Jörn
<120> Mittel zur Prävention und/oder Behandlung einer Gewebeveränderung mesenchymalen Ursprungs
<130> B3960PCT
<140>
   <141>
<160> 47
<170> PatentIn Ver. 2.1
<210> 1
   <211> 19
   <212> DNA
   <213> Künstliche Sequenz
<220>
   <223> Beschreibung der künstlichen Sequenz:Primer HsgA1
<400> 1
   aaggtgtcaa tyatgtttg                                    19
<210> 2
   <211> 14
   <212> DNA
   <213> Künstliche Sequenz
<220>
   <223> Escchreibung der künstlichen Sequenz:Primer HsgA2
<400> 2
   acggttactt kttt                                         14
<210> 3
   <211> 18
   <212> DNA
   <213> Künstliche Sequenz
<220>
   <223> Beschreibung der künstlichen Sequenz:Primer HsgB1
<400> 3
   tctattccct acctggat 18
<210> 4
   <211> 17
   <212> DNA
   <213> Künstliche Sequenz
<220>
   <223> Beschreibung der künstlichen Sequenz:Primer HsgB2
<400> 4
   actcttaacg gcagtag 17
<210> 5
   <211> 32
   <212> DNA
   <213> Künstliche Sequenz
<220>
   <223> Beschreibung der künstlichen Sequenz:Primer ADE1Bg12S
<400> 5
   gaagatcttt atagatggaa tggtgccaac at 32
<210> 6
   <211> 31
   <212> DNA
   <213> Künstliche Sequenz
<220>
   <223> Beschreibung der künstlichen Sequenz:Primer ADE1Hi3AS
<400> 6
   cccaagctta aaactcttct cgctggcagt c 31
<210> 7
   <211> 17
   <212> DNA
   <213> Künstliche Sequenz
<220>
   <223> Beschreibung der künstlichen Sequenz:Primer HsgC1
<400> 7
   acttttgact cttctgt 17
<210> 8
   <211> 17
   <212> DNA
   <213> Künstliche Sequenz
<220>
   <223> Beschreibung der künstlichen Sequenz:Primer HsgC2
<400> 8
   tccttgtatt tagtatc 17
<210> 9
   <211> 18
   <212> DNA
   <213> Künstliche Sequenz
<220>
   <223> Beschreibung der künstlichen Sequenz:Primer HsgD1
<400> 9
   ccatcatgtt cgactcct 18
<210> 10
   <211> 17
   <212> DNA
   <213> Künstliche Sequenz
<220>
   <223> Beschreibung der künstlichen Sequenz: Primer HsgD2
<400> 10
   aggtagccgg tgaagcc 17
<210> 11
   <211> 18
   <212> DNA
   <213> Künstliche Sequenz
<220>
   <223> Beschreibung der künstlichen Sequenz: Primer HsgE1
<400> 11
   gactcttccg tcagctgg 18
<210> 12
   <211> 17
   <212> DNA
   <213> Künstliche Sequenz
<220>
   <223> Beschreibung der künstlichen Sequenz: Primer HsgE2
<400> 12
   gctggtaacg gcgctct 17
<210> 13
   <211> 17
   <212> DNA
   <213> Künstliche Sequenz
<220>
   <223> Beschreibung der künstlichen Sequenz: Primer HsgF1
<400> 13
   atttctattc cttcgcg 17
<210> 14
   <211> 17
   <212> DNA
   <213> Künstliche Sequenz
<220>
   <223> Beschreibung der künstlichen Sequenz: Primer HsgF2
<400> 14
   tcaggcttgg tacggcc 17
<210> 15
   <211> 430
   <212> DNA
   <213> Adenovirus: Isolat X765551Ko
<400> 15
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccaatgt aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccataccaac acaacaactc tggctttgta 360
   gggtatcttg cacctactat gagacaaggg gaaccttacc cagccaatta tccatacccg 420
   ctcatcggaa 430
<210> 16
   <211> 430
   <212> DNA
   <213> Adenovirus: Isolat M2-3s
<400> 16
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccaatgt aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccataccaac acaacaactc tggctttgta 360
   gggtatcttg cacctactat gagacaaggg gaaccttacc cagccaatta tccatacccg 420
   ctcatcggaa                                                        430
<210> 17
   <211> 430
   <212> DNA
   <213> Adenovirus: Isolat M7-1s
<400> 17
   ggcaccttct accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag gatacaacgt ggcacaatgc aacatgacca aagactggtt cctagttcag 180
   atgcttgcca actacaacat tggctaccag ggcttttaca tccctgaggg atacaaggat 240
   cgcatgtact cttttttcag aaacttccag cctatgagca ggcaggtggt tgatgaggtt 300
   aattacactg actacaaagc cgtcacctta ccataccaac acaacaactc tggctttgta 360
   gggtaccttg cacctactat gagacaaggg gaaccttacc cagccaatta tccatacccg 420
   ctcatcggaa
<210> 18
   <211> 430
   <212> DNA
   <213> Adenovirus: Isolat M8-2s
<400> 18
   ggcaccttct accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag gatacaacgt ggcacaatgc aacatgacca aagactggtt cctagttcag 180
   atgcttgcca actacaacat tggctaccag ggcttttaca tccctgaggg atacaaggat 240
   cgcatgtact cttttttcag aaacttccag cctacgagca ggcaggtggt tgatgaggtt 300
   aattacactg actacaaagc cgtcacctta ccataccaac acaacaactc tggctttgta 360
   gggtaccttg cacctactat gagacaaggg gaaccttacc cggccaatta tccatacccg 420
   ctcatcggaa
<210> 19
   <211> 143
   <212> PRT
   <213> Adenovirus: Isolat X765551Ko
<400> 19
   Gly Thr Phe Tyr Leu Asn His Thr Phe Lys Lys Val Ser Ile Met Phe
   1               5                   10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Gln Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Lys His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 20
   <211> 143
   <212> PRT
   <213> Adenovirus: Isolat M2-3s
<400> 20
   Gly Thr Phe Tyr Leu Asn His Thr Phe Lys Lys Val Ser Ile Met Phe
    1               5                  10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Gln Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Lys His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 21
   <211> 143
   <212> PRT
   <213> Adenovirus: Isolat M7-1s
<400> 21
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Met Ser Arg Gln Val
   85 90 95
   Val Asp Glu Val Asn Tyr Thr Asp Tyr Lys Ala Val Thr Leu Pro Tyr
   100 105 110
   Lys His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 22
   <211> 143
   <212> PRT
   <213> Adenovirus: Isolat M8-2s
<400> 22
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Thr Ser Arg Gln Val
   85 90 95
   Val Asp Glu Val Asn Tyr Thr Asp Tyr Lys Ala Val Thr Leu Pro Tyr
   100 105 110
   Lys His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 23
   <211> 430
   <212> DNA
   <213> Adenovirus: Isolat AF065068Ko
<400> 23
   ggcacttttt accttaacca cactttcaag aaggtctcca tcatgtttga ctcctcagtc 60
   agctggcctg gcaatgacag gctgttgtct ccaaatgagt ttgaaatcaa gcgcactgtg 120
   gatggggaag gatacaatgt ggcccaatgc aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccatatcaac acaacaactc tggctttgta 360
   ggataccttg cgcctactat gagacaaggg gaaccttacc cagccaatta tccatacccg 420
   ctcatcggaa 430
<210> 24
   <211> 430
   <212> DNA
   <213> Adenovirus: Isolat M6-1s
<400> 24
   ggcacctttt accttaacca cactttcaag aaggtctcca tcatgtttga ctcctcagtc 60
   agctggcctg gcaatgacag gctgttgtct ccaaatgagt ttgaaatcaa gcgcactgtg 120
   gatggggaag gatacaatgt ggcccaatgc aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccatatcaac acaacaactc tggctttgta 360
   ggataccttg cgcctactat gagacaaggg gaaccttacc cagccaatta tccatacccg 420
   ctcatcggaa 430
<210> 25
   <211> 143
   <212> PRT
   <213> Adenovirus: Isolat AF065068Ko
<400> 25
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Lys Gly Phe Tyr Ile Pro Glu Gly Tyr Lys Asp
   65 70 75 80
   Arg Met Tyr Ser Phe Phe Arg Asn Phe Gln Pro Met Ser Arg Gln Val
   85 90 95
   Val Asp Glu Val Asn Tyr Thr Asp Tyr Lys Ala Val Thr Leu Pro Tyr
   100 105 110
   Lys His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 26
   <211> 143
   <212> PRT
   <213> Adenovirus: Isolat M6-1s
<400> 26
   Gly Thr Phe Tyr Leu Asn His Thr Phe Lys Lys Val Ser Ile Met Phe
   1               5                   10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Lys Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Lys His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 27
   <211> 430
   <212> DNA
   <213> Adenovirus: Isolat AF065065Ko
<400> 27
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccaatgt aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccataccaac acaacaactc tggctttgta 360
   gggtatcttg cacctactat gagacaaggg gaaccttacc cagccaatta tccatacccg 420
   ctcatcggaa 430
<210> 28
   <211> 430
   <212> DNA
   <213> Adenovirus: Isolat M3.3P-2
<400> 28
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccanngt aacatgacca aagactggtt cctggttcag 180
   atgcttgcca actacaacat tggctaccag ggcttttaca tncctgaggg atacaaggat 240
   cgcatgtact cctttttcag aaacttccag cctatgagca ggcaggtggc tgatgaggnt 300
   aattacactg actacaaagc cggcacctta ccataccaac acaacaactc tggctttgta 360
   gggtatcttg cacctactat gagacaaggg gaaccttacc cagccaatta tccatacccg 420
   ctcatcggaa                                                        430
<210> 29
   <211> 430
   <212> DNA
   <213> Adenovirus: Isolat M5-1s
<400> 29
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccaatgt aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccataccaac acaacaactc tggctttgta 360
   gggtatcttg cacctactat gagacaaggg gaaccttacc cagccaatta tccatacccg 420
   ctcatcggaa                                                        430
<210> 30
   <211> 430
   <212> DNA
   <213> Adenovirus: Isolat M9-2s
<400> 30
   ggcaccttct accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag gatacaacgt ggcacaatgc aacatgacca aagactggtt cctagttcag 180
   atgcttgcca actacaacat tggctaccag ggcttttaca tccctgaggg atacaaggat 240
   cgcatgtact cttttttcag aaacttccag cctatgagca ggcaggtggt tgatgaggtt 300
   aattacactg actacaaagc cgtcacctta ccataccaac acaacaactc tggctttgta 360
   gggtaccttg cacctactat gagacaaggg gaaccttacc cagccaatta tccatacccg 420
   ctcatcggaa                                                        430
<210> 31
   <211> 143
   <212> PRT
   <213> Adenovirus: Isolat AF065065Ko
<400> 31
   Gly Thr Phe Tyr Leu Asn His Thr Phe Lys Lys Val Ser Ile Met Phe
    1               5                  10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Gln Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Gln His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 32
   <211> 143
   <212> PRT
   <213> Adenovirus: Isolat M3-3p
<400> 32
   Gly Thr Phe Tyr Leu Asn His Thr Phe Lys Lys Val Ser Ile Met Phe
    1               5                  10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Xaa Xaa Asn Met Thr Lys Asp Trp Phe Leu Val Gln Met Leu Ala Asn
       50                  55                  60
   Tyr Asn Ile Gly Tyr Gln Gly Phe Tyr Xaa Pro Glu Gly Tyr Lys Asp
   65                  70                  75                      80
   Arg Met Tyr Ser Phe Phe Arg Asn Phe Gln Pro Met Ser Arg Gln Val
                   85                  90                  95
   Ala Asp Glu Xaa Asn Tyr Thr Asp Tyr Lys Ala Gly Thr Leu Pro Tyr
               100                 105                 110
   Gln His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
           130                 135                 140
<210> 33
   <211> 143
   <212> PRT
   <213> Adenovirus: Isolat M5-1s
<400> 33
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Met Ser Arg Gln Val
   85 90 95
   Val Asp Glu Val Asn Tyr Thr Asp Tyr Lys Ala Val Thr Leu Pro Tyr
   100 105 110
   Gln His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 34
   <211> 143
   <212> PRT
   <213> Adenovirus: Isolat M9-2s
<400> 34
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Met Ser Arg Gln Val
   85 90 95
   Val Asp Glu Val Asn Tyr Thr Asp Tyr Lys Ala Val Thr Leu Pro Tyr
               100                 105                 110
   Gln His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 35
   <211> 430
   <212> DNA
   <213> Adenovirus: Isolat M2-3s
<400> 35
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccaatgt aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccataccaac acaacaactc tggctttgta 360
   gggtatcttg cacctactat gagacaaggg gaaccttacc cagccaatta tccatacccg 420
   ctcatcggaa                                                        430
<210> 36
   <211> 430
   <212> DNA
   <213> Adenovirus: Isolat M5-1s
<400> 36
   ggcacctttt accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag ggtacaatgt ggcccaatgt aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccataccaac acaacaactc tggctttgta 360
   gggtatcttg cacctactat gagacaaggg gaaccttacc cagccaatta tccatacccg 420
   ctcatcggaa
<210> 37
   <211> 430
   <212> DNA
   <213> Adenovirus: Isolat M6-1s
<400> 37
   ggcacctttt accttaacca cactttcaag aaggtctcca tcatgtttga ctcctcagtc 60
   agctggcctg gcaatgacag gctgttgtct ccaaatgagt ttgaaatcaa gcgcactgtg 120
   gatggggaag gatacaatgt ggcccaatgc aacatgacca aagactggtt cctggttcag 180
   atgcttgcca actacaacat tggctaccag ggcttttaca tccctgaggg atacaaggat 240
   cgcatgtact cctttttcag aaacttccag cctatgagca ggcaggtggt tgatgaggtt 300
   aattacactg actacaaagc cgtcacctta ccatatcaac acaacaactc tggctttgta 360
   ggataccttg cgcctactat gagacaaggg gaaccttacc cagccaatta tccatacccg 420
   ctcatcggaa
<210> 38
   <211> 430
   <212> DNA
   <213> Adenovirus: Isolat M7-1s
<400> 38
   ggcaccttct accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag gatacaacgt ggcacaatgc aacatgacca aagactggtt cctagttcag 180
   atgcttgcca actacaacat tggctaccag ggcttttaca tccctgaggg atacaaggat 240
   cgcatgtact cttttttcag aaacttccag cctatgagca ggcaggtggt tgatgaggtt 300
   aattacactg actacaaagc cgtcacctta ccataccaac acaacaactc tggctttgta 360
   gggtaccttg cacctactat gagacaaggg gaaccttacc cagccaatta tccatacccg 420
   ctcatcggaa
<210> 39
   <211> 430
   <212> DNA
   <213> Adenovirus: Isolat M8-2s
<400> 39
   ggcaccttct accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag gatacaacgt ggcacaatgc aacatgacca aagactggtt cctagttcag 180
   atgcttgcca actacaacat tggctaccag ggcttttaca tccctgaggg atacaaggat 240
   cgcatgtact cttttttcag aaacttccag cctacgagca ggcaggtggt tgatgaggtt 300
   aattacactg actacaaagc cgtcacctta ccataccaac acaacaactc tggctttgta 360
   gggtaccttg cacctactat gagacaaggg gaaccttacc cggccaatta tccatacccg 420
   ctcatcggaa
<210> 40
   <211> 430
   <212> DNA
   <213> Adenovirus: Isolat M9-2s
<400> 40
   ggcaccttct accttaacca cactttcaag aaggtctcca tcatgtttga ctcctcagtc 60
   agctggcctg gcaatgacag gctgttgagc ccaaatgagt ttgaaatcaa gcgcactgtg 120
   gacggggaag gatacaacgt ggcacaatgc aacatgacca aagactggtt cctagttcag 180
   atgtttgcca actacaacat tggctaccag ggcttttaca tccctgaggg atacaaggat 240
   cgcatgtact cttttttcag aaacttccag cctatgagca ggcaggtggt tgatgaggtt 300
   aattacactg actacaaagc cgtcacctta ccataccaac acaacaactc tggctttgta 360
   gggtaccttg cacctactat gagacaaggg gaaccttacc cagccaatta tccatacccg 420
   ctcatcggaa
<210> 41
   <211> 143
   <212> PRT
   <213> Adenovirus: Isolat M2-3s
<400> 41
   Gly Thr Phe Tyr Leu Asn His Thr Phe Lys Lys Val Ser Ile Met Phe
   1               5                   10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Gln Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Lys His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 42
   <211> 143
   <212> PRT
   <213> Adenovirus: Isolat M5-1s
<400> 42
   Gly Thr Phe Tyr Leu Asn His Thr Phe Lys Lys Val Ser Ile Met Phe
   1               5                   10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Gln Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Lys His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 43
   <211> 143
   <212> PRT
   <213> Adenovirus: Isolat M6-1s
<400> 43
   Gly Thr Phe Tyr Leu Asn His Thr Phe Lys Lys Val Ser Ile Met Phe
   1               5                   10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Met Ser Arg Gln Val
   85 90 95
   Val Asp Glu Val Asn Tyr Thr Asp Tyr Lys Ala Val Thr Leu Pro Tyr
   100 105 110
   Lys His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 44
   <211> 143
   <212> PRT
   <213> Adenovirus: Isolat M7-1s
<400> 44
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Met Ser Arg Gln Val
   85 90 95
   Val Asp Glu Val Asn Tyr Thr Asp Tyr Lys Ala Val Thr Leu Pro Tyr
   100 105 110
   Lys His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 45
   <211> 143
   <212> PRT
   <213> Adenovirus: Isolat M8-2s
<400> 45
   Gly Thr Phe Tyr Leu Asn His Thr Phe Lys Lys Val Ser Ile Met Phe
   1 5 10 15
   Asp Ser Ser Val Ser Trp Pro Gly Asn Asp Arg Leu Leu Ser Pro Asn
   20 25 30
   Glu Phe Glu Ile Lys Arg Thr Val Asp Gly Glu Gly Tyr Asn Val Ala
   35 40 45
   Gln Cys Asn Met Thr Lys Asp Trp Phe Leu Val Gln Met Leu Ala Asn
   50 55 60
   Tyr Asn Ile Gly Tyr Gln Gly Phe Tyr Ile Pro Glu Gly Tyr Lys Asp
   65 70 75 80
   Arg Met Tyr Ser Phe Phe Arg Asn Phe Gln Pro Thr Ser Arg Gln Val
   85 90 95
   Val Asp Glu Val Asn Tyr Thr Asp Tyr Lys Ala Val Thr Leu Pro Tyr
   100 105 110
   Lys His Asn Asn Ser Gly Phe Val Gly Tyr Leu Ala Pro Thr Met Arg
   115 120 125
   Gln Gly Glu Pro Tyr Pro Ala Asn Tyr Pro Tyr Pro Leu Ile Gly
   130 135 140
<210> 46
   <211> 143
   <212> PRT
   <213> Adenovirus: Isolat M9-2s
<400> 46
   Gly Thr Phe Tyr Leu Asn His Thr Phe Lys Lys Val Ser Ile Met Phe
   1               5                   10                  15
   Asp Ser Ser Val Ser Trp Pro Gly Asn Asp Arg Leu Leu Ser Pro Asn
               20                  25                  30
   Glu Phe Glu Ile Lys Arg Thr Val Asp Gly Glu Gly Tyr Asn Val Ala
           35                  40                  45
   Gln Cys Asn Met Thr Lys Asp Trp Phe Leu Val Gln Met Leu Ala Asn
       50                  55                  60
   Tyr Asn Ile Gly Tyr Gln Gly Phe Tyr Ile Pro Glu Gly Tyr Lys Asp
   65                  70                  75                  80
   Arg Met Tyr Ser Phe Phe Arg Asn Phe Gln Pro Met Ser Arg Gln Val
                   85                  90                  95
   Val Asp Glu Val Asn Tyr Thr Asp Tyr Lys Ala Val Thr Leu Pro Tyr
               100                 105                 110
   Lys His Asn Asn Ser Gly Phe Val Gly Tyr Leu Ala Pro Thr Met Arg
           115                 120                 125
   Gln Gly Glu Pro Tyr Pro Ala Asn Tyr Pro Tyr Pro Leu Ile Gly
       130                 135                 140
<210> 47
   <211> 720
   <212> DNA
   <213> Adenovirus
<223> Promotorsequenz des adenoviralen Proteins E1A (wie dargestellt in Fig. 5)
<400> 47
   ctctctatat aatatacctt atagatggaa tggtgccaac atgtaaatga ggtaatttaa 60
   aaaagtgcgc gctgtgtggt gattggctgt ggggtgaatg actaacatgg gcggggcggc 120
   cgtgggaaaa tgacgtgact tatgtgggag gagttatgtt gcaagttatt gcggtaaatg 180
   tgacgtaaaa ggaggtgtgg tttgaacacg gaagtagaca gttttcccac gcttactggt 240
   aggatatgag gtagttttgg gcggatgcaa gtgaaaattc tccattttcg cgcgaaaact 300
   gaatgaggaa gtgaatttct gagtcatttc gcggttatga cagggtggag tatttgccga 360
   gggccgagta gactttgacc gtttacgtgg aggtttcgat taccgtgttt ttcacctaaa 420
   tttccgcgta cggtgtcaaa gtcctgtgtt tttacgtagg tgtcagctga tcgctagggt 480
   atttaaacct gacgagttcc gtcaagaggc cactcttgag tgccagcgag aagagttttc 540
   tcctccgcgc cgcaagtcag ttctgcgctt tgaaaatgag acacctgcgc ttcctgccac 600
   aggagattat ctccagtgag accgggatcg aaatactgga gtttgtggta aataccctaa 660
   tgggagacga cccggaaccg ccagtgcagc cttttgatcc acctacgctg cacgatctgt 720

## Beschreibung

Die Erfindung betrifft die Verwendung eines Mittels zur Prävention und/oder Behandlung einer Gewebeveränderung, wobei die Gewebeveränderung Gewebe mesenchymalen Ursprungs umfaßt und die Verwendung von Verfahren zur Diagnose einer Gewebeveränderung, wobei die Gewebeveränderung eine Gewebeveränderung mesenchymalen Ursprungs ist.

Die Behandlung von Gewebeveränderungen wie Tumoren und Karzinomen ist trotz intensivster Bemühungen der modernen Medizin noch immer vergleichsweise beschränkt auf Chemotherapie in ihren vielfältigsten Abwandlungen und chirurgische Eingriffe. Bei weitem problematischer ist die Prävention derartiger Gewebeerkrankungen. In der Regel gilt es dabei, die für die Gewebeveränderung verantwortlichen Faktoren für den individuellen Lebensbereich auszuschließen, was jedoch nicht immer möglich ist.

Ein besonderer Problemkreis ist die Prävention und Behandlung von Gewebeveränderungen mit mesenchymalem Ursprung. Bis zum heutigen Tage ist die der so verschiedenen Tumoren wie Leiomyom, Endometrium-Polyp, Endometriose, Hamartom der Lunge und der Mamma, Prostata-Adenom, Atherom und vieler mehr, zugrundeliegende Ätiologie nicht bekannt, was dazu führt, daß für diese Art der Erkrankungen nur eine symptomatische Therapie bleibt. Unter diesen Umständen ist eine Prävention ganz besonders schwierig, weiß doch der Einzelne nicht, wie er durch sein eigenes Verhalten das Risiko, an einer derartigen Gewebeveränderung zu erkranken, verringern kann.

Beispielhaft sei hier auf die Leiomyome des Uterus hingewiesen. Diese sind, obwohl sie gutartig ausgebildet sind, ein gesundheitspolitisches Problem. So haben Studien ergeben, daß in den westeuropäischen Staaten jede dritte Frau Uterusleiomyome aufweist und bspw. in den USA jeder fünfte Besuch beim Gynäkologen aufgrund von Myomen erfolgt (Morton, C.C.; Am. J. Pathol. 1998; 153(4): 1050-20). Wenn diese Leiomyome sich symptomatisch äußern, kann dies z. B. mit erheblichen Blutungen und damit gesundheitlichen Risiken, Schmerzen und Harninkontinenz verbunden sein. Darüber hinaus können diese Leiomyome Infertilität bei den betroffenen Frauen zur Folge haben.

Trotz vielfältiger Bemühungen war die Ätiologie/Pathogenese von Uterusleiomyomen bisher unklar (Morton aaO.). Verschiedene mögliche Ursachen von Uterus-Leiomyomen werden bspw. von Cramer, S.F. et al. (Cramer S.F.; J. Reprod. Med. 1995, 40(8): 595-600) diskutiert, so bspw. Verletzung und Reparatur des Endometriums. Auch die Beteiligung von Östrogen und/oder Progesteron und Östrogen-und/oder Progesteron-Rezeptoren bei der Entstehung von Neoplasmen der glatten Muskulatur des Uterus wurde in der Literatur diskutiert, so z. B. von Tiltman A.J. (Tiltman A.J.; Curr. Opin. Obstet. Gynecol., 1997; 9(1):48-51. Aufgrund von verschiedenen Untersuchungen hat sich jedoch die Vorstellung entwickelt, daß Mutationen der Gene der HMGI(Y)-Familie daran beteiligt sind (siehe Schoenmakers E.F. et al.; Nat. Genet. 1995, 10(4):436-44), wobei nach wie vor ungeklärt ist, ob es sich bei den besagten Mutationen um primäre oder sekundäre Ereignisse handelt.

Trotz dieser Erkenntnis stehen für die Myome derzeit nur zwei Therapieansätze zur Verfügung, zum einen die operative Entfernung der Gebärmutter (Hysterektomie), so alleine in den USA pro Jahr ca. 200.000 Hysterektomien (Morton, aaO), oder die Myomenukleation, d.h. das "Herausschälen" der Tumoren, bei Uterus-Erhaltung. Im Falle der Myomenukleation kann präoperativ eine medikamentöse Myomverkleinerung herbeigeführt werden unter Verwendung von Hormon-Antagonisten, die jedoch insoweit mit unerwünschten Nebenwirkungen verbunden sind, als daß dadurch menopausale Beschwerden ausgelöst werden.

Es besteht somit für Leiomyome, insbesondere solche des Uterus, sowie Endometriumpolypen und Endometriose ein dringender Bedarf an neuen Konzepten für Therapie und Präventionen und den hierfür geeigneten Mitteln.

Es ist somit eine Aufgabe der vorliegenden Erfindung, Mittel zur Prävention und Behandlung von Gewebeveränderungen bereitzustellen, wobei die Gewebeveränderung Gewebe mesenchymalen Ursprungs umfaßt.

Des weiteren liegt der Erfindung die Aufgabe zugrunde, ein Verfahren zur Diagnose einer Gewebeveränderung bereitzustellen, wobei die Gewebeveränderung Gewebe mesenchymalen Ursprungs umfaßt,

Die Aufgabe wird gelöst durch den Gegenstand des beigefügten unabhängigen Ansprüche. Bevorzugte Ausfürungsformen ergeben sich aus den beigefügten Unteransprüchen.

Bevor im folgenden die Erfindung näher erläutert wird, sollen die hierin verwendeten Begrifflichkeiten in Ergänzung zu dem allgemeinen Verständnis des Fachmannes auf dem Gebiet definiert werden.

Unter Leiomyomen sollen hierin insbesondere benigne Tumoren der glatten Muskulatur verstanden werden (Definition, nach Baltzer, J. et al.; Gynäkologie - Ein kurzgefaßtes Lehrbuch, 5. Aufl., 1994, Thieme Verlag).

Unter Endometriumpolypen sollen hierin insbesondere Hyperplasien und polypöse Wachstumsformen des Endometriums mit stromaler und Drüsen-(epithelialer) oder ausschließlich stromaler Komponente verstanden werden.

Unter Endometriose sollen hierin insbesondere Endometriumherde, die sich an anderer Stelle als im Cavum uteri (Definition nach Baltzer aaO.) befinden, verstanden werden.

Unter Myomen sollen hierin insbesondere Leiomyome verstanden werden (Definition nach Baltzer aaO.).

Der Erfindung liegt ganz allgemein die überraschende Erkenntnis zugrunde, daß Gewebeveränderungen, die ein Gewebe umfassen, das mesenchymalen Ursprungs ist, ein gemeinsamer Pathogenitätsmechanismus zugrunde liegt. Der , mit der Anwesenheit eines Virus in dem Gewebe mesenchymalen Ursprungs verbunden ist, wobei das Virus ein solches ist, dessen Nukleinsäure mindestens eine Bindungsstelle für ein Genprodukt von Genen der HMGI(Y)-Familie oder deren Derivate umfaßt oder das Virus ein solches ist, dessen Nukleinsäure für ein Genprodukt codiert, wobei dieses Genprodukt mindestens mit einem Genprodukt von Genen der HMGI(Y)-Familie oder deren Derivate wechselwirkt und das Virus ausgesgewählt ist aus der Gruppe, die Adenoviren, Herpesviren und Papovariren enthält.. Diese Viren werden hierin auch als "HMGI-Viren" bezeichnet oder Bezug genommen durch den Begriff "wie hierin beschrieben". Mit anderen Worten, diese Art von Viren sind ein Faktor bei der Auslösung von Gewebeveränderungen, die Gewebe umfassen, das mesenchymalen Ursprungs ist.

Im Zusammenhang damit scheinen innerhalb der Gruppe der DNA-Viren insbesondere die Adenoviren hierfür von besonderer Bedeutung zu sein. Eine weitere Gruppe von DNA-Viren, die für die hierin beschriebenen Gewebeveränderungen, die ein Gewebe umfassen, das mesenchymalen Ursprungs ist, von besonderer Bedeutung sind, sind Viren der Herpesgruppe, die im folgenden und hierin vereinfacht als Herpesviren bezeichnet werden. Desweiteren gehören zur Gruppe der DNA-Viren, die für die hierin beschriebenen Gewebeveränderungen von Bedeutung sind, die Papova-Viren.

Infolge der vorstehend beschriebenen Erkenntnisse konnte der Erfinder die erfindungsgemäßen Mittel schaffen. Sind Viren an den Gewebeveränderungen in der einen oder anderen Form kausal beteiligt, kann diese Kausalität und damit die Entstehung, Aufrechterhaltung und/oder Weiterentwicklung der Gewebeveränderungen durch antivirale Mittel beeinflußt werden. Ein antivirales Mittel kann damit, vereinfacht ausgedrückt, gegen Gewebeveränderungen eingesetzt werden, die von einem Virus, wie es hierin beschrieben ist, d.h. HMGI-Viren und/oder DNA-Viren, infiziert bzw. verursacht werden. Gleichzeitig ergibt sich daraus, daß bei derartigen Gewebeveränderungen besonders jene antiviralen Mittel verwendet werden können, die wirksam sind gegen die hierin beschriebenen Viren, d.h. die hierin als HMGI-Viren und DNA-Viren bezeichneten Viren, insbesondere deshalb und soweit sie an der Gewebeveränderung direkt oder indirekt kausal beteiligt sind.

Die insbesondere in den Ansprüchen verwendete Begrifflichkeit "ein Virus nach einem der vorangehenden Ansprüche" greift somit auf die Beschreibung der Viren, wie sie in einem der Ansprüche gegeben wird, zurück, ohne daß die Viren jedoch u. U. als solches beansprucht sind, um nicht nochmals deren Merkmale zu wiederholen. Im allgemeinen wird durch diese verkürzte Schreibweise Bezug genommen auf HMGI-Viren und/oder DNA-Viren, wie sie hierin beschrieben sind.

Die hierin beschriebenen Gewebeveränderungen können Tumore und/oder Karzinome umfassen. Hinsichtlich des histologischen Aufbaus der besagten Gewebeveränderungen kann festgestellt werden, daß diese vollständig aus solchem Gewebe bestehen können, das sich unter dem Einfluß der HMGI-Viren und/oder DNA-Viren (insbesondere Adenoviren und/oder Herpesviren), die, sofern sich aus dem jeweiligen Kontext nichts anderes ergibt, hierin auch als "die besagten Viren" insgesamt bezeichnet werden, verändert hat, oder aber ein Teil des die Gewebeveränderung aufweisenden Gewebes aus solchem Gewebe besteht, das sich unter dem Einfluß der besagten Viren verändert hat, mithin dieser Teil des Gewebes lediglich ein, aber ein obligater Bestandteil der Gewebeveränderung oder Tumors ist.

Die Gewebeveränderung kann des weiteren eine solche sein, bei der die Veränderung auf eine Proliferation von mit den besagten Viren infizierten mesenchymalen Zellen zurückzuführen ist. Gleichzeitig kann die Gewebeveränderung auch eine epitheliale Komponente aufweisen. Unter epithelialer Komponente soll hierin ein Teil der Gewebeveränderung (bspw. des Tumors oder Karzinoms) verstanden werden, der hinsichtlich seines histologische Ursprungs dem Epithel zugewiesen werden kann. Diese histologische Zuweisung erfolgt dabei auf der Basis allgemein akzeptierter Kriterien der Histopathologie.

Bei den Gewebeveränderungen kann dabei auch die Situation vorliegen, daß die Proliferation eine klonale Proliferation ist, d.h. die Proliferation auf ein einzelnes Infektionsereignis zurückgeht, mithin eine einzelne Zellen infolge der Infektion mit den besagten Viren transformiert wird. Infolge der Transformation dieser einzelnen Zelle verändert sich das Verhalten der Zelle, insbesondere ihr Differenzierungszustand und/oder ihr Wachstumsverhalten, was zu der Gewebeveränderung führt. Geht die Gewebeveränderung auf eine einzelne, mit den besagten Viren infizierte Zellen zurück, spricht man von einem monoklonalen Tumor, geht die Gewebeveränderung auf mehrere aber wenige mit den besagten Viren infizierte Zellen zurück, spricht man von einem oligoklonalen Tumor. Beide Klonalitäten gehen auf den hierin offenbarten durch die besagten Viren vermittelten Pathogenitätsmechanismus zurück.

Mit der Infektion der mesenchymalen Zellkomponente von Gewebe durch die besagten Viren, das schließlich die Gewebeveränderung zeigt bzw. diese ausbildet, als universellen Pathogenitätsmechanismus sind eine Reihe von verschiedenen Infektionsszenarien möglich. So kann, z. B., in einem Fall die Primärinfektion in einem Gewebe mesenchymalen Ursprungs erfolgen und auf dieses beschränkt sein. Dabei kann es zu weiteren Gewebeveränderungen kommen, wobei eine Epithelproliferation auftritt, die mittelbar oder unmittelbar Folge der Virusinfektion der mesenchymalen Komponente ist. Beispiele hierfür stellen Hamartome und Endometriose dar. Es ist jedoch auch möglich, daß sich die Primärinfektion auf andere Gewebeteile ausweitet. Dabei kann bspw. auch epitheliales Gewebe infiziert werden, wobei es sich dabei um eine Sekundärinfektion handelt und dieses sekundär infizierte epitheliale Gewebe ebenfalls proliferieren kann. Es ist des weiteren denkbar, daß epitheliales Gewebe zuerst, d.h. primär, durch die besagten Viren infiziert wird und sich ausgehend hiervon eine Sekundärinfektion des mesenchymalen Gewebes oder Gewebeanteils der - späteren - Gewebeveränderung ergibt, die dann zu einer Proliferation des mesenchymalen Gewebes führen kann. Ein Beispiel für das zuletzt beschriebene Szenario stellt das Kolonadenom dar.

Die hierin beschriebenen Viren können während oder nach der Infektion in der Zelle in irgendeiner Form vorliegen. So kann das Virus in den infizierten Zellen episomal oder in das Wirtsgenom integriert vorliegen. Es kann einen lytischen Zyklus durchlaufen, in die Umgebung freigesetzt werden und weitere Zellen infizieren, wie auch oben ausgeführt. Das Virus kann auch in epithelialen Zellen entweder episomal oder in das Genom integriert vorliegen.

Unter Adenovirus soll hierin ganz allgemein ein jegliches Mitglied der Gruppe der Adenoviren verstanden werden, wie sie bspw. beschrieben ist in Fields Virology, 3 rd Edition, Raven Publisher, Philadelphia, 1996 und die dort beschriebenen Eigenschaften aufweist. Gleiches gilt für die Viren aus der Gruppe der Herpesviren, d. h. unter Herpesvirus soll hierin ganz allgemein ein jegliches Virus aus der Gruppe der Herpesviren verstanden werden, z. B. auch das Cytomegalovirus, wie in Fields Virology, a.a.O., beschrieben und das die dort beschriebenen Eigenschaften aufweist. Des weiteren erstreckt sich der Begriff der Adenoviren und/oder Herpesviren hierin insbesondere im Zusammenhang mit dem gegen Adenoviren und/oder Herpesviren gerichteten Impfstoff, auch auf solche Viren, die wesentliche Elemente und/oder Eigenschaften dieser Viren aufweisen. Hierzu zählen bspw. auch gentechnologisch veränderte Viren.

Zur Gruppe der DNA-Viren gehören auch die Polyoma-Viren, die wiederum zur Familie der Papovaviridae, zu denen auch die Papilloma-Viren und Simian Vacuolating Virus 40 (SV 40) gehören. Die Familie zeichnet sich durch extreme Hitzestabilität aus. Papovaviridae sind hüllenlose kubische DNA-Viren mit einem Durchmesser von 45 bis 55 nm, umfassend 72 Kapsomere sowie eine zyklische doppelsträngige DNA. Das hierin insbesondere zu bzw. im Zusammenhang mit Adenoviren und Herpesviren Gesagte gilt sinngemäß auch für diese Gruppe der DNA-Viren.

Infolge dieses insbesondere beim Menschen hinsichtlich Gewebeveränderungen, die Gewebe, das mesenchymalen Ursprungs ist, umfassen, nach derzeitiger Auffassung des Erfinders, universellen Pathogenitätsmechanismus, der auf der Beteiligung der besagten, d. h. hierin beschriebenen Viren beruht, besteht damit die Möglichkeit, Mittel bzw. Therapiekonzepte zur Behandlung von all jenen Gewebeveränderungen bereitzustellen, die Gewebe mesenchymalen Ursprung umfassen. Gleiches gilt für die Diagnose derartiger Gewebeveränderungen.

Darüberhinaus kann in Kenntnis dieses Pathogenitätsmechanismus nun auch eine Prävention unter Verwendung der erfindungsgemäßen Mittel betrieben werden. Die Umsetzung dieser fundamentalen Erkenntnis besteht darin, die hierin beschriebenen antiviralen Mittel zur Behandlung derartiger Gewebeveränderungen zu verwenden. Diese Mittel können insoweit auch bereits bekannte antiviral wirksame Mittel umfassen, solange sie geeignet sind, die Aktivität der hierin beschriebenen Viren, d.h. HMGI-Viren und/oder DNA-Viren, zu beeinflussen. Als möglichen Angriffspunkt für eine derartige Beeinträchtigung der viralen Aktivität sind sämtliche Teilschritte bzw. Teilaspekte denkbar, einschließlich des Absorptionsvorganges, Intemalisierungsvorganges, Integration der viralen DNA in das Wirtsgenom oder Stabilisierung im Zytoplasma der Wirtszelle, Replikation, Transkription und Translation, Zusammenbau des Viruskapsids und Freisetzung des Viruskapsids. Daneben eignen sich auch besonders Impfstoffe gegen diese Viren sowohl zur Therapie, besonders jedoch zur Prävention derartiger Gewebeveränderungen, wie im folgenden detaillierter ausgeführt werden wird.

Beispielhaft seien in der nachfolgenden Tabelle 1 Gewebeveränderungen angeführt, für die die erfindungsgemäßen Mittel jeweils angewandt werden können bzw. für deren Therapie und Prävention die bereits bekannten antiviralen Mittel, wie sie beispielsweise in Tabelle 2 gezeigt sind, verwendet werden können.

**Tabelle 1**

| **Gewebeveränderung/ Tumor** | **Mesenchymale Komponente** | **Epitheliale Komponente** | **Enstehung maligner Tumoren** |
|---|---|---|---|
| Leiomyome (Uterus) | wie glatte Muskulatur, monoklonal, ca. 15% Mutationen der HMGI(Y)-Gene | fehlt | Möglichkeit der sar-omatösen Um-andlung fraglich; wenn überhaupt sehr selten |
| Endometrium-Polypen | Stroma, monoklonal, ca. 45% Mutationen der HMGI(Y)-Gene | polyklonal | Karzinomentstehung aus der epithelialen Komponente |
| Endometriose | vorhanden (Stroma) | vorhanden | wird nicht diskutiert |
| Fibroadenome (Mamma) | Stroma, klonale Chromosomenveränderungen beschrieben, ca. 10% Mutationen der HMGI(Y)-Gene | Drüsenepithel | Möglichkeit der Karzinomentstehung aus der epithelialen Komponente fraglich; wenn überhaupt sehr selten |
| Phyllodes-Tumoren (Mamma) | Stroma, klonale Chromosomenveränderungen / Mutationen der HMGI(Y)-Gene beschrieben | Drüsenepithel | teilweise maligne |
| Hamartome (Mamma) | wie Fettgewebe, monoklonal, Mutationen der HMGI(Y)-Gene beschrieben | Drüsenepithel | Möglichkeit der Karzinomentstehung aus der epithelialen Komponente fraglich; wenn überhaupt sehr selten |
| Prostata-Adenome | wie glatte Muskulatur (Synonym: Adenomyome), klonale Chromosomenveränderungen beschrieben | Drüsenepithel | Enstehung von Prostata-Karzinomen |
| Lipome | Wie Fettgewebe, monoklonal, ca. 40% Mutationen der HMGI(Y)-Gene | fehlt | praktisch keine Umwandlung zum Liposarkom |
| Aggressive Angiomyxome | Myxoid, klonale Chromosomenveränderungen (Zielgen: *HMGIC)* | fehlt | charakteristisch: weitlumige Gefässe |
| Enchondrome | Wie hyaliner Knorpel, klonale Chromosomenveränderungen beschrieben (u.a.12q14-15) | fehlt | praktisch keine Umwandlung zum Chondrosarkom |
| Pleomorphe Adenome (Kopfspeicheldrüsen) | mesenchymale und gemeinsamer klonaler wird angenommen, | epitheliale Komponente, Ursprung beider Anteile ca. 20% *HMGIC-Mut.* | Enstehung von Karzinomen *ex pleomorphic adenoma* |
| Kolon-Polypen (Kolon-Adenome) | Stroma | Darmepithel | Adenom-Karzinom-Sequenz histologisch gesichert |
| Hamartome (Lunge) | Wie hyaliner Knorpel, Fettgewebe, glatte Muskulatur, monoklonal, ca. 70% Mutatio nen der HMGI(Y)-Gene | epitheliale Spalten (Bronchialepithel) | Möglichkeit der Karzinomentstehung aus der epithelialen Komponente fraglich; wenn überhaupt sehr selten |
| Atherome | u.a. wie glatte Muskulatur, klonale Chromosomenveränderungen beschrieben | Endothelüberzug | wird nicht diskutiert |

**Tabelle 2: Übersicht über verschiedene antivirale Verbindungen, die im Rahmen der vorliegenden Erfindung verwendet werden können.**

| **Wirkstoff** | **Handelsname (beispielhaft)** | **Wirkweise** |
|---|---|---|
| Adenosinarabinosid | Vidarabin | Hemmung der viralen Replikation |
| Bromvinyluridinarabinosid | Brovavir, Sorivudin | Hemmung der viralen Replikation |
| 9-(1,3-Dihydroxy-2-propoxy) methylguanin | Gabciclovir | Hemmung der viralen Polymerase |
| Trinatriumsalz der Phosphonoameisensäure | Foscamet | Hemmung der viralen Polymerase |
| Interferon α | Roferon A | Hemmung der viralen Proteinsysnthese |
| Interferon β | Fiblaferon | Hemmung der viralen Proteinsynthese |
| Interferon γ | Imukin | Hemmung der viralen Proteinsynthese |
| Immunglobulin | Pentaglobin | Antikörper vermittelte antivirale Wirkung |

Neben dem vorstehend geschilderten universellen Pathogenitätsmechanismus hat der Erfinder weiterhin überraschend festgestellt, daß die Entstehung der Gewebeveränderungen, die Gewebe mesenchymalen Ursprungs umfassen bzw. entsprechender Tumore, aufbauend auf der oben beschriebenen Infektion mit den besagten Viren durch ein zusätzliches Ereignis in synergistischer Weise gefördert wird. Bei diesem zusätzlichen Ereignis handelt es sich um chromosomale Veränderungen, insbesodere um solche, die die HMGI(Y) - Gene betreffen, d.h. Bruchpunkte struktureller Chromosomenaberrationen liegen entweder innerhalb der Gene oder in einer Entfernung von diesen, so daß es durch die strukturellen Chromosomenaberrationen zu einer transkriptionellen De-Regulierung der HMGI(Y)-Gene kommt. Infolge dieser chromosomalen Veränderung kommt es zur Expression nunmehr veränderter zellulärer Genprodukte, die mit bestimmten viralen Sequenzen interagieren, wodurch der beobachtete synergistische Effekt bei der Genese der Gewebeveränderungen bedingt wird. Der Begriff der HMGI(Y)-Gene wird im folgenden im Zusammenhang mit den als erfindungsgemäße Mittel offenbarten Impfstoffen noch weiter ausgeführt werden.

Beispiele von Gewebeveränderungen, die neben der Infektion des mesenchymalen Gewebeanteils noch eine chromosomale Veränderung der HMGI(Y) aufweisen ergeben sich aus der obige Tabelle 1, wobei beispielhaft auf Endometriumpolypen, Endometrioseherde, Hamartome der Lunge, Lipome, Fibroadenome der Mamma und pleomorphe Adenome der Speicheldrüsen verwiesen wird. Wie auch insbesondere aus Tabelle 1 ersichtlich, kann der Anteil derjenigen Gewebeveränderungen, die neben der Infektion durch die besagten Viren, d.h. HMGI-Viren und/oder DNA-Viren, noch eine chromosomale Veränderung der HMGI(Y)-Gene tragen, schwanken. Der Nachweis der chromosomalen Veränderung ist beispielsweise beschrieben in Kazmierczak et. al., Oncogene 12: 515 - 521.

Wie bereits vorstehend erwähnt, setzt die Prävention und Therapie mesenchymaler Gewebeveränderungen, die Gewebe mesenchymalen Ursprungs umfassen, bei einer antiviralen Therapie an. Die Gewebeveränderungen können jedoch neben der viralen Infektion noch chromosomale Veränderung betreffend die HMGI(Y)-Gene aufweisen. Auch derartige Gewebeveränderungen können, da auch sie letzten Endes auf einer viralen Aktivität beruhen, mit den hierin vorgeschlagenen Mitteln behandelt bzw. verhindert werden. Damit werden auch die eingangs beschriebenen Untersuchungen von Schoenmakers (a.a.O.) betreffend Mutationen der Gene der HMGI(Y)-Familie an Uterus-Leiomyomen erklärlich, wenngleich aus dem darin geschilderten Befund, die hierin offenbarte technische Lehre nicht abgeleitet werden konnte. Die Pathogenese der Leiomyome beruht eben nicht nur auf Mutationen, insbesondere Chromosomenaberrationen im Bereich der Loci von Mitgliedern der HMGI(Y)-Genfamilie, sondern es handelt sich, zumindest bei einigen dieser Fälle, d. h. Gewebeveränderung unter Beteiligung eines Gewebes mesenchymalen Ursprungs mit einer Mutation im Bereich der Gene der HMGI(Y)-Gene, um eine Folge aus dem Zusammenwirken von Mutationsereignissen der Mitglieder der HMGI(Y)-Genfamilie und einem Virus, insbesondere eines transformierenden Virus, bzw. Infektion mit einem solchen handelt, wobei die virale Infektion selbst bereits ausreicht, um eine Myomentstehung auszulösen, dessen Wachstumspotential durch eine - ggf. nachfolgende - Mutation von Genen der HMGI(Y)-Familie verstärkt wird.

Ohne im folgenden durch diese Annahmen beschränkt sein zu wollen, insbesondere nicht im Detail, scheint es derzeit so zu sein, daß infolge der Infektion mit einem transformierenden Virus sowie dessen folgender Persistenz, mutmaßlich nach Integration in das zelluläre Genom, in Zellen ohne weitere Mutation von Genen der HMGI(Y)-Familie die transformierenden viralen Proteine, ggf. nur schwach, exprimiert werden und die resultierenden Tumoren daher sehr langsam wachsen und klein bleiben. Infolge einer mutationsbedingten ReAktivierung von Genen der HMGI(Y)-Familie, z. B. durch Verlagerung von Enhancem in Juxta-Position zu den Genen, kommt es zu einer verstärkten Aktivierung der Gene der transformierenden Proteine und einer insgesamt erhöhten Expression derselben. Die in der Zelle vermehrt vorhandenen transformierenden Proteine führen zu einer deutlich höheren Wachstumsaktivität der entsprechenden Tumoren und somit zu einem verstärkten Tumorwachstum.

Konkret hat man gefunden, daß die Genprodukte der Mitglieder der Gene der HMGI(Y)-Familie, die typischerweise an Nukleinsäuren binden können, so bspw. beschrieben von French, S.W. et al. (French, S.W. et al.; Mol. Cell Biol., 1996; 16(10): 5393-99; Yie, J. et al.; Mol. Cell Biol., 1997, 17(7): 3649-62), auch an Nukleinsäure von transformierenden Viren binden, wobei bei Bindung im Bereich der regulatorischen Regionen der Nukleinsäure (z.B. Promotoren und Enhancer) die Genprodukte der Gene der HMGI(Y)-Familie auf die Transkriptionsrate der viralen transformierenden Proteine Einfluß nehmen.

Weiterhin hat man gefunden, daß die Genprodukte der Mitglieder der Gene der HMGI(Y)-Familie auch mit einem Genprodukt einer viralen Nukleinsäure wechselwirken können.

Diese Wechselwirkung kann in einer direkten Wechselwirkung der beiden Genprodukte, d.h. dem Genprodukt der viralen Nukleinsäure und demjenigen eines Gens der HMGI(Y)-Familie, bestehen. Eine andere Form der Wechselwirkung kann darin bestehen, daß diese durch eine weitere Komponente vermittelt wird, d.h. keine direkte Wechselwirkung der beiden Genprodukt-Spezies vorliegt. Diese vermittelnde weitere Komponente kann bspw. eine Nukleinsäure sein, und insbesondere eine solche Nukleinsäure, die eine Bindungsstelle für ein Genprodukt von Genen der HMGI(Y)-Familie aufweist, wie sie oben beschrieben wurde.

Infolge dieses Zusammenwirkens von Genprodukten der Gene der HMGI(Y)-Familie mit Nukleinsäure von transformierenden Viren, insbesondere den regulatorischen Regionen der Nukleinsäure (z.B. Promotoren und Enhancer) dieser Viren, kann der Entstehung von Gewebeveränderungen mit Gewebe mesenchymalen Ursprungs, wie sie beispielhaft in Tabelle 1 oben angegeben sind, dadurch entgegengewirkt werden, daß ein antivirales Mittel gegen die Viren verwendet wird. Wie vorstehend ausgeführt, sind allgemein derartige antivirale Mittel zu diesem Zweck geeignet, die gegen die an der Gewebeveränderung kausal beteiligten Viren gerichtet, d. h. aktiv sind. Die Viren sind die hierin beschriebenen Viren, d.h. HMGI-Viren und/oder DNA-Viren. Eine - bevorzugte - Untergruppe der hierin beschriebenen Viren sind solche Viren, bei denen das einzelne Virus eine Nukleinsäure aufweist bzw. für diese codiert, die mindestens eine Bindungsstelle für ein Genprodukt von Genen der HMGI(Y)-Familie oder deren Derivate umfaßt oder die für ein Genprodukt codiert, wobei das Genprodukt mindestens mit einem Genprodukt von Genen der HMGI(Y)-Familie oder deren Derivate wechselwirkt, und die zur Gruppe der DNA-Viren gehören.

Im Zusammenhang damit scheinen innerhalb der Gruppe der DNA-Viren insbesondere die Adenoviren hierfür von besonderer Bedeutung zu sein. Eine weitere Gruppe von DNA-Viren, die für die hierin beschriebenen Gewebeveränderungen, die ein Gewebe umfassen, das mesenchymalen Ursprungs ist, von besonderer Bedeutung sind, sind Viren der Herpesgruppe, die im folgenden vereinfacht als Herpesviren bezeichnet werden. Desweiteren gehören zur Gruppe der DNA-Viren, die für die hierin beschriebenen Gewebeveränderungen von Bedeutung sind, die Papova-Viren. Der vorliegenden Erfindung liegt weiterhin die Erkenntnis zugrunde, daß für die hierin beschriebenen Gewebeveränderungen verschiedene Viren, insbesondere DNA-Viren, synergistisch wirken können.

Die erfindungsgemäß Verwendeten antiviralen Mittel, die gegen die hierin beschriebenen Viren gerichtet bzw. wirksam sind, zu denen auch gegen diese Viren gerichtete Impfstoffe gehören, verhindern somit, daß die entsprechenden Gewebe, viral infiziert werden bzw. es zu einer Vermehrung des viralen Materials kommt und somit die Ausbildung des Komplexes aus viraler Nukleinsäure und Genprodukten der Gene der HMGI(Y)-Familie verhindert wird, was in der Folge dazu führt, daß die Ausbildung der Gewebeveränderungen unterbleibt.

Bei der Pathogenese der hierin beschriebenen Gewebeveränderungen scheint weiterhin eine Bindung von Genprodukten viraler Nukleinsäure mit Genprodukten von Genen der HMGI(Y)-Familie und deren Derivaten zu erfolgen und infolge dessen sich der Einfluß der viralen transformierenden Proteine zu erhöhen. Infolge der verschiedenen antiviralen Mittel wird dieses Zusammenwirken letztendlich verhindert oder zumindest verringert.

Hierin soll Bindung eines Genprodukts von Genen der HMGI(Y)-Familie oder deren Derivaten an virale Nukleinsäure oder an das Genprodukt viraler Nukleinsäure insbesondere eine jegliche Wechselwirkung der daran beteiligten Molekülspezies umfassen, die u.U. dazu führen kann, daß die den Komplex ausbildenden Komponenten nicht mehr als Einzelkomponenten wahrgenommen werden, sondern der Komplex die einzig wahrnehmbare Komponente ist, was jedoch nicht ausschließt, daß Einzelkomponenten noch in nicht-gebundener Form vorhanden sind. Eine derartige Bindung schließt bspw. Wechselwirkung durch elektrostatische Anziehungskräfte, van der Wals-Kräfte, hydrophobe Wechselwirkung, Wasserstoffbrückenbindungen und Disulfidbrücken und Kombinationen davon ein. Ein derartiger Komplex umfaßt zumindest die beiden Genproduktespecies, d.h. ein Genprodukt der viralen Nukleinsäure und ein Genprodukt der Gene der HMGI(Y)-Familie, die direkt miteinander.wechselwirken können, kann aber auch zusätzlich eine Nukleinsäure umfassen, wobei auch in diesem Fall eine direkte Wechselwirkung der beiden Genproduktspecies erfolgen kann, jedoch nicht notwendigerweise erfolgen muß.

Insoweit betrifft ein Aspekt der Erfindung auch die Verwendung eines Impfstoffes gegen transformierende Viren, der zur Prävention und/oder Behandlung der hierin beschriebenen Gewebeveränderungen geeignet ist.

Grundsätzlich sind alle der hierin offenbarten Mittel sowohl zur Therapie als auch zur Prävention von Gewebeveränderungen der hierin beschriebenen Art geeignet. Ganz besonders vorteilhaft für die Prävention sind dabei solche Mittel, die einen Impfstoff gegen die hierin beschriebenen Viren, d.h. HMGI-Viren und/oder DNA-Viren, umfassen, die somit die bisherigen unbefriedigenden Therapie- und Präventionskonzepte mit ihren nicht unerheblichem Gefährdungspotential obsolet machen. Dabei ist besonders beachtlich, daß die Verwendung von Impfstoffen für den Körper besonders schonend, da mit in der Regel vergleichsweise weniger Nebenwirkungen verbunden, ist.

Die Herstellung von Impfstoffen gegen Viren ist in der Technik allgemein bekannt und bspw. beschrieben in Modrow, S.; Falke, D.; Molekulare Virologie, Spektrum, Akad. Verl., 1997.

Bei Impfstoffen sind grundsätzlich verschiedene Arten bekannt, nämlich Lebendimpfstoffe, die vermehrungsfähiges Virus enthalten, Impfstoffe aus inaktiviertem Virus, wobei die Viren in nicht mehr vermehrungsfähiger Form vorliegen, Spaltimpfstoffe, die nur aus den für die Immunisierung wichtigen Komponenten des Virus bestehen, auch als Subunit-Vakzine oder Antigenimpfstoffe bezeichnet, und sogenannte "synthetische Antigenimpfstoffe". Alle vorerwähnten Impfstofformen können im Rahmen der vorliegenden Erfindung verwendet werden.

Lebendimpfstoffe enthalten vermehrungsfähige Virusstämme, die einen spezifischen Schutz bewirken, aber bei gesunden Tieren zu keiner Erkrankung führen. Ein Lebendimpfstoff kann entweder aus homologen (artgleichen) oder heterologen (artfremden) Virusstämmen hergestellt werden. Als homologe Impfstoffe können natürlich gewonnene oder künstlich hergestellte Stämme eines Virus verwendet werden.

Natürlich gewonnene Impfvirusstämme stammen von Feldstämme ab, die nur noch eine schwache bzw. keine Virulenz mehr besitzen, also bei gesunden Tieren keine Erkrankung mehr hervorrufen, sich aber im Wirt vermehren und die Ausbildung einer Immunität bewirken.

Eine Untergruppe der Lebendimpfstoffe betrifft die künstlich abgeschwächten, attenuierten Stämme. Sie werden aus gut immunisierenden, vollvirulenten Feldviren dadurch gewonnen, daß sie nach einer mehr oder weniger großen Anzahl von Passagen, bevorzugt in Zellkulturen, künstlich kultiviert werden, wodurch sie ihre Virulenz für den natürlichen Wirt verlieren. Der Virulenzverlust in einer derartigen passierten Viruspopulation tritt nicht gleichzeitig bei allen Viruspartikeln ein. Durch bestimmte Selektionsverfahren kann man aber attenuierte Viruspartikel isoliert gewinnen (z. B. Plaque-Methode, Endverdünnungsmethode usw.).

Unter Attenuierung versteht man eine gezielte Abschwächung oder Aufhebung der Virulenz eines vermehrungsfähigen Virus für einen bestimmten Wirt unter Erhaltung der Vermehrungsfähigkeit, der Antigenität und der Immunogenität, die über eine bestimmte Generationsfolge konstant bleibt.

Die Attenuierung kann eine Modifikation oder eine Mutation hinsichtlich Verlust der krankmachenden Eigenschaften, hier im vorliegenden Falle insbesondere hinsichtlich der transformierenden Eigenschaften, sein. Sie ist entsprechend mehr oder weniger stabil. Während man in früherer Zeit für eine künstliche Virulenzabschwächung vorwiegend Passagen in Tieren oder Bruteiern durchfährte, gewinnt man heute attenuierte Stämme hauptsächlich aus Zellkulturen über Dauerpassagen.

Lebendvakzine aus heterologen Virusstämmen können dann einen Immunschutz bewirken, wenn zwischen artverschiedenen Viren sehr enge immunologische Verwandtschaftsbeziehungen bestehen. Man kann dann als Impfstamm den verwandten heterologen und deshalb nicht krankmachenden Virusstamm verwenden.

Lebendvakzine haben Vor- und Nachteile. Durchschnittlich wird mit ihnen eine bessere und länger anhaltende Immunität erzielt. Ein gut attenuiertes Impfvirus kann den Immunitätsmechanismus nur dann stimulieren, wenn es in ausreichender Konzentration verabreicht wird. Die Bestimmung einer entsprechenden ausreichenden Konzentration kann durch einfache routinemäßige Versuche erfolgen, die im Rahmen des Könnens des Durchschnittfachmanns liegen. Der Impfschutz setzt in der Regel schon nach wenigen Tagen nach der Impfung ein. Verantwortlich sind hierfür mehrere Vorgänge: Interferenz, Interferon-Bildung, schnelle Entwicklung einer zellulären Immunität. Ein weiterer Vorteil von Lebendvakzinen besteht darin, daß sie sehr leicht lokal appliziert werden können, z. B. oral oder per Aerosol. Dies ist besonders mit Blick auf die vergleichsweise leichte Zugänglichkeit der betroffenen Gewebe bzw. Organe im Falle der hierin beschriebenen Gewebeveränderungen vorteilhaft, da in einem solchen Fall der Endverbraucher ein entsprechendes Mittel selbst anwenden kann.

Weiterhin können gemäß der vorliegenden Erfindung Impfstoffe aus inaktivierten Viren verwendet werden. Als Virusinaktivierung wird generell die Aufhebung der Infektiosität eines Virusteilchens bezeichnet. In der Impfstoffherstellung speziell versteht man unter Inaktivierung, daß Viren künstlich, mit chemisch-physikalischen Verfahren, die Vermehrungsfähigkeit genommen wird, ohne daß dabei die anderen Aktivitäten, insbesondere die antigenen und immunogenen Fähigkeit negativ beeinflußt werden. Für Impfstoffe aus inaktivierten Viren findet sich in der Literatur teilweise noch der Begriff "antigener Impfstoff".

Als Ausgangsmaterial für diese Impfstoffe dienen aus virushaltigen Organen oder Geweben, heute jedoch in erster Linie aus Zellkulturen hergestellte, gereinigte und hochkonzentrierte Virussuspensionen von vollvirulenten gut immunisierenden Virusstämmen. Diese konzentrierten Virussuspensionen werden dann schonend durch geeignete Verfahren inaktiviert. Optimal sind chemische oder physikalische Behandlungen, die die Virusnukleinsäure als Träger der Vermehrungsfähigkeit und Infektiosität zerstören, jedoch die Proteinanteile des Virus, die wirksame Komponenten für Antigenität und Immunogenität sind, möglichst wenig schädigen, denaturieren oder verändern. Bewährte Mittel sind z. B. Formaldehyd und bestimmte Detergenzien. Als physikalische Komponenten verwendet man Wärme und Strahlen.

Weiterhin können im Zusammenhang mit der vorliegenden Erfindung Spaltimpfstoffe verwendet werden, die durch Aufspaltung von Viren gewonnene antigene und immunisierende Viruskomponenten in, in der Regel, gereinigter Form enthalten.

Bei den behüllten Viren sind dies die virusspezifischen Glycoproteide der lipidhaltigen Hüllen. Spezifische Antikörper gegen diese Antigene binden in vivo an die Glycoproteide des Virus, blockieren dadurch deren Haftungsfunktion an die Zellen und damit die Infektiosität.

Die immunisierenden Glycoproteide behüllter Viren kann man durch die chemische Aufspaltung der Virushülle (bestimmte lipidlösende Detergenzien) freisetzen. Das Virus verliert dadurch spontan und restlos seine infektiösen Eigenschaften und anschließend wird das gewünschte Glycoproteid von unerwünschten Komponenten wie Nukleoprotein, Kapsidenzym, Lipide etc. des aufgespaltenen Virusmaterials durch physikalisch-chemische Verfahren gereinigt, konzentriert und zum Impfstoff verarbeitet.

Bei unbehüllten, nackten Viren sind Spaltimpfstoffe aus den für die Immunisierung maßgeblichen Kapsidproteinen von den Fachleuten auf dem Gebiet entwickelbar.

Schließlich können auch synthetische antigene Impfstoffe verwendet werden, die mittels gentechnologischer Verfahren hergestellt werden. Über Isolierung und Identifizierung der maßgeblichen Gene des Virusgenoms kann beispielsweise das Kapsidprotein der entsprechenden Viren gentechnologisch produziert werden. Dabei ist es im Rahmen der Fähigkeiten des Fachmannes die entsprechende Nukleinsäure bzw. daraus resultierenden Proteine soweit zu verkürzen, daß lediglich die antigene Determinante bzw. das entsprechende Epitop als Impfstoffkomponente verbleibt.

Den Impfstoffen können Adjuvanzien hinzugesetzt werden, wie bspw. vollständiges oder unvollständiges Freundsches Adjuvans. Weitere Zusätze sind den Fachleuten auf diesem Gebiet bekannt.

Allgemein können die erfindungsgemäß verwendeten Mittel als pharmazeutisches Präparat vorliegen, das neben mindestens einem der verschiedenen erfindungsgemäßen Mittel auch einen geeigneten pharmazeutisch akzeptablen Träger umfaßt. Geeigenete pharmazeutisch akzeptable Träger sind den Fachleuten bekannt.

Die erfindungsgemäß verwendeten Mittel können weitere Zusätze, die bspw. der Stabilisierung des Mittels, der Konservierung, der Modifikation der Eigenschaften des Mittels selbst dienen, sowie Stoffe, die die Eigenschaften der erfindungsgemäßen Mittel modifizieren, umfassen.

Derartige Stoffe, die die Eigenschaften des erfindungsgemäße verwendeten Mittels selbst modifizieren, können bspw. im Falle von Impfstoffen Adjuvanzien, wie bspw. unvollständiges oder vollständiges Freundsches Adjuvans, sein.

Die erfindungsgemäß verwendeten Mittel können weiterhin Komponenten aufweisen, die sie in eine für die jeweilige angestrebte Applikationsform bevorzugte Form überführen. So kann z. B. im Falle der Ausbildung der erfindungsgemäß verwendeten Mittel lals Aerosole neben dem eigentlichen Mittel ein für die Aerosolbildung erforderliches Trägermaterial vorgesehen sein.

Das erfindungsgemäß verwendete Mittel kann bspw. in Form einer Lösung oder Emulsion zur intradermalen, intravenösen oder subkutanen Injektion vorliegen. Flüssige Lösungen der erfindungsgemäße verwendeten Mittel können auch in infundierbarer Form vorliegen.

Weiterhin können die erfindungsgemäß verwendeten Mittel in lyophilisierter Form vorliegen.

Darüberhinaus können die erfindungsgemäß verwendete, Mittel grundsätzlich in einer jeglichen pharmazeutisch geeigneten Form vorliegen, einschließlich, bspw., Tabletten, Dragees, Suppositorien, Gele, Pulver.

Für den Fall, daß das erfindungsgemäß verwendete Mittel einen Impfstoff umfaßt, kann der Impfstoff selbst ein vollständiges Viruspartikel sein, lebend oder attenuiert, ebenso wie inaktiviert oder Teile davon, wobei die Teile davon typischerweise die antigenen und immunologischen Eigenschaften des jeweiligen Virus tragen. Es kann dabei vorgesehen sein, daß der Impfstoff eine Vielzahl verschiedener Viruspartikel oder antigener bzw. immunogen wirksamer Teile umfaßt. Die Viruspartikel sowie die Teile können selbst in modifizierter Form vorliegen. Derartige Modifikationen können ausgebildet sein durch, wie dies allgemein für Peptide, Proteine, die ggf. glycosyliert sind, bekannt ist.

Die Viruspartikel oder Teile davon können gentechnologisch hergestellt sein. Es ist nicht erforderlich, daß die Viruspartikel oder Teile davon mit den für die Entstehung der hierin beschriebenen Gewebeveränderungen verantwortlichen oder zumindest damit assoziierten Viren identisch ist. Entscheidend ist, daß die im Impfstoff verwendeten oder zu dessen Herstellung verwendeten Viren, einschließlich Teile davon, geeignet sind, eine gegen das für die Erkrankung kausale Agens, d.h. Virus, gerichtete Immunantwort zu erzeugen und somit die transformierenden Eigenschaften des an der Pathogenese beteiligten Agens bzw. Virus zu verhindern.

Das vorstehend Gesagte gilt sinngemäß auch für den Fall, daß der Impfstoff einen Antikörper umfaßt.

Man hat gefunden, daß die Bindungsstelle für ein Genprodukt von Genen der HMGI(Y)-Familie oder deren Derivate auf der Nukleinsäure des Virus eine Reihe von Struktur- und Sequenzmerkmalen umfaßt. Grundsätzlich scheint für die Bindung von Genprodukten der HMGI(Y)-Familie, Teilen davon und deren Derivaten, das Vorhandensein einer - ersten - AT-reichen Sequenz von Bedeutung zu sein. Dabei ist AT-reiche Sequenz nicht so zu verstehen, daß dies eine ausschließliche Abfolge bestehend aus AT-Dimeren ausbildet. Vielmehr kann diese Sequenz eine beliebige Reihenfolge der beiden Basen umfassen, die durch einzelne oder mehrere Nukleotide unterbrochen sein kann. Neben diesem ersten Struktur- und Sequenzmerkmal weisen die besagten Bindungsstellen der Genprodukte der HMGI(Y)-Familie noch eine zweite AT-reiche Sequenz auf, die ähnlich ausgebildet sein kann wie die erste AT-reiche Sequenz. Beide AT-reiche Sequenzen sind in einer räumlichen Distanz relativ zueinander angeordnet. Diese räumliche Distanz ergibt sich aus den räumlichen Abmessungen und damit aus der Sekundär- und Tertiärstruktur der HMGI(Y)-Proteine, d.h. der Genprodukte der Gene der HMGI(Y)-Familie. Infolge dieser Sekundär- und Tertiärstruktur ist für ein Binden des HMGI(Y)-Genproduktes erforderlich, daß die erste Sequenz und die zweite Sequenz relativ zueinander in einer Ebene auf der viralen Nukleinsäure angeordnet sind. Betrachtet man die DNA als dreidimensionales Modell, so liegen bei der vorgeschilderten Anordnung die als Bindungsstellen wirkenden AT-reichen Sequenzen auf der dem Betrachter jeweils zugewandten Seite. Diese Anordnung wird auch als "same face" bezeichnet. Liegen diese drei Struktur- bzw. Sequenzmerkmale der viralen Nukleinsäure vor, kommt es zu einem besonders nachhaltigen Binden des Genprodukts an die virale Sequenz, insbesondere in regulatorischen Regionen der Nukleinsäure (z.B. Promotoren und Enhancer), da bevorzugt diese die besagten Struktur- und Sequenzmerkmale aufweist. Fehlt eines oder mehrere dieser Sequenz- bzw. Strukturmerkmale, wird die Bindung eines Genproduktes der Gene der HMGI(Y)-Familie nur schwach oder gar nicht erfolgen.

Betrachtet man umgekehrt die Sekundär- bzw. Tertiärstruktur der Genprodukte der Gene der HMGI(Y)-Familie, so existieren dort drei Bereiche, oder Domänen, die in einer definierten Entfernung voneinander angeordnet sind, die zu AT-reichen Sequenzen eine - hohe - Affinität aufweisen. Eine erfolgreiche Bindung an eine Nukleinsäure setzt somit voraus, daß die AT-reichen Sequenzen in einer hierzu korrespondierenden Entfernung angeordnet sind. Diese Entfernung resultiert aus der Ganghöhe der Nukleinsäure, die in der Regel ca. 10 Basenpaare beträgt, woraus resultiert, daß der Abstand der AT-reichen Sequenzen in der Regel 10 Basenpaare sowie ganzzahlige Vielfache bis zu ≤ 3 davon beträgt.

Gemäß der vorliegenden Erfindung sind neben Impfstoffen allgemein, besonders solche Impfstoffe gegen die hierin beschriebenen Viren, d.h. HMGI-Viren und/oder DNA-Viren, solche Impfstoffe für die erfindungsgemäßen Mittel geeignet, die gegen ein vorstehend genanntes Virus gerichtet sind, dessen Nukleinsäure mindestens eine Bindungsstelle für mindestens ein Genprodukt von Genen der HMGI(Y)-Familie oder deren Derivate aufweist. Gene der HMGI(Y)-Familie sind in der Technik gut bekannt.

Die Gene der HMGI(Y)-Familie stellen eine Familie von Genen dar, die, unter anderem, HMGIC, HMGIY und MAG-Gene umfaßt. Beispielhaft sei hierzu auf die internationale Patentanmeldungen PCT/EP96/00716 und PCT/DE96/02494 beschrieben, deren Offenbarungsgehalt hierin durch Bezugnahme aufgenommen wird. Beachtlich ist, daß die Bindungsstelle auf der viralen Nukleinsäure für ein beliebiges der vorstehend genannten Gene bzw. dessen Genprodukt geeignet ist. Hierin soll der Begriff Genprodukt auch Teile davon bezeichnen, solange diese Teile noch geeignet sind, eine virale Nukleinsäure zu binden. Gleichfalls soll der Begriff Genprodukt die jeweiligen Derivate des Genproduktes umfaßen, die eine Modifikation aufweisen, wie sie üblicherweise für Peptide und Proteine vorgenommen werden kann, und bspw. Deletionen und Austausche der carboxyterminalen Abschnitte der Proteine umfaßt. Insbesondere umfaßt der Begriff Derivate der Gene der HMGI(Y)-Familie auch die in PCT/DE96/02494 beschriebenen aberranten Transkripte sowie deren Translationsprodukte, solange diese nach wie vor in der Lage sind, an die virale Nukleinsäure, insbesondere in regulatorischen Regionen der Nukleinsäure (z.B. Promotoren und Enhancer) derselben, zu binden. Die Charakterisierung derartiger aberranter Transkripte ist bspw. beschrieben von Kazmierczak, B. et al; Am. J. Pathol., 1998; 153(2):431-5 und deren Struktur bspw. von Schoenmakers, E. F. et al, aaO.

In einer alternativen Ausführungsform der erfindungsgemäß verwendeten Mittel kann vorgesehen sein, daß der Impfstoff ein Antikörper ist, der gegen das Virus bzw. zumindest einen der hierin beschriebenen Viren gerichtet ist. Es ist dabei ausreichend, wenn der Antikörper nicht primär gegen das Virus oder einen entsprechenden Teil davon gerichtet ist, sondern vermittels einer entsprechenden Kreuzreaktivität seine Wirkung erfüllt und entsprechend gewährleistet, daß es zu keiner Wechselwirkung zwischen der viralen Nukleinsäure mit dem Genprodukt der Gene der HMGI(Y)-Familie in dem Sinne kommt, daß der oben beschriebene Pathogenitätsmechanismus greift und es zu den beschriebenen Gewebeveränderungen kommt. Dabei kann vorgesehen sein, daß der Antikörper gegen eine jede beliebige Komponente des Virus gerichtet ist, d.h. er kann gerichtet sein gegen bzw. wechselwirken mit Proteinen oder Proteinfragmenten des Viruskapsids, des gegebenenfalls vorhanden Glycopeptids oder aber auch mit der entsprechenden viralen Nukleinsäure bzw. Fragmenten davon.

Der Antikörper, wie er im Rahmen der Erfindung verwendet wird, kann ein monoklonaler Antikörper oder polyklonaler Antikörper sein. Der Begriff Antikörper kann hierin eine Mischung verschiedener monoklonaler Antikörper umfassen. Weiterhin soll hierin der Begriff Antikörper ein jegliches Peptid oder Protein umfassen, welches mindestens eine Antikörpereigenschaft aufweist, insbesondere an ein geeignetes Epitop bindet und dafür sorgt, daß der Komplex aus Antikörper plus Epitop bzw. Antigen in einer für die Pathogenese der hierin beschriebenen Gewebeveränderungen nicht mehr zugängliche Form überführt wird bzw. aus dem Pathogeneseprozeß entfernt wird. Dies kann bspw. dadurch erfolgen, daß eine Wechselwirkung eines Genproduktes eines Gens der HMGI(Y)-Familie mit der viralen Nukleinsäure oder eine solche mit einem Genprodukt einer viralen Nukleinsäure (wobei das entsprechende Virus - kausal - an der Entstehung der hierin beschriebenen Gewebeveränderungen beteiligt ist) letztenendes vermieden wird, wobei diese Verhinderung sowohl direkt als auch indirekt bedingt werden kann, indirekt bspw. durch Entfernen der entsprechenden Viren bzw. Virenpartikel. Der Begriff Antikörper schließt auch Antikörperfragmente und deren Derivate ein, so insbesondere (Fab)' oder F(ab)₂-Fragmente sowie Einzelketten-Antikörper und dgl. Derivate dieser Antikörper sind den Fachleuten ebenfalls bekannt und schließen insbesondere ein.

Das vorstehend Gesagte gilt sinngemäß auch für den Fall, daß ein Impfstoff gegen ein Virus vorgesehen ist, dessen Nukleinsäure für ein Genprodukt codiert, wobei dieses Genprodukt mindestens mit einem Genprodukt von Genen der HMGI(Y)-Familie oder deren Derivate wechselwirkt.

Die Viren, gegen die ein Impfstoff bzw. ein Antikörper in den erfindungsgemäßen Mittel enthalten ist, gehören zu den verschiedenen Gruppen der hierin beschriebenen Viren mit ihren verschiedenen Typen.

Bei der erfindungsgemäßen Verwendung des Mittels zur Immunisierung, und insbesondere aktiven Immunisierung, gegen die Viren, die mit der Pathogenese und/oder Ätiologie der hierin beschriebenen Gewebeveränderungen verbunden sind, sind bevorzugt die Viren jene, die kausal mit der Pathogenese/Ätiologie der hierin beschriebenen Gewebeveränderungen verbunden bzw. assoziiert sind.

Unter normalem Karyotyp soll hierin verstanden werden der Chromosomensatz, wie er nach Anwendung routinemäßiger Techniken erhalten wird, sofern er bei einer Darstellung von ≥ 500 Banden pro haploidem Satz keine erkennbaren Anomalien insbesondere der Bereiche 12q14-15 und 6p21 aufweist.

Die Kontrollkulturen sind in der Regel jene mit normalem Karyotyp, die abgeleitet sind von einem Gewebe oder Teil eines Gewebes, welches in einer hierin beschriebenen Gewebeveränderung enthalten ist, und welche mit einem Expressionsvektor transfiziert sind, dem jedoch das Gen der HMGI(Y)-Familie oder dessen Derivat, d.h. das für ein Genprodukt codierende Insert, fehlt. Kontrollkulturen können aber auch solche sein, die mit keinerlei Expressionsvektor transfiziert sind.

Bei einem weiteren erfindungsgemäßen Verfahren, bei dem die Durchführung eines PCR-Tests vorgesehen ist, wobei die für die PCR-verwendete Sonde eine virale Sonde ist, erfolgt der PCR-Test nach den in der Technik bekannten Verfahren. Unter PCR-Test wird ein Polymerase-Kettenreaktion-Test verstanden, bei dem mindestens ein sequenzspezifischer Primer zur selektiven Amplifikation der gesuchten Nukleinsäure bzw. des gesuchten Nukleinsäurefragmentes verwendet wird. (siehe Newton, C.R.; Graham, A.; "PCR"; 1994, Spektrum Akadem. Verlag, Heidelberg, Berlin, Oxford.

Unter Primer oder viraler Sonde sollen hierin Oligonukleotide und/oder Nukleinsäurefragmente verstanden werden, die gerichtet sind auf eine Detektion von Nukleinsäuren, die eine Homologie zu dem Primer/der Sonde aufweisen. Die Herstellung derartige viraler Sonden kann auf allen den Fachleuten bekannte Wegen erfolgen, so bspw. durch organische chemische Synthese oder unter Verwendung von PCR oder Klonierungstechniken.

Wird im Rahmen eines erfindungsgemäßen Verfahrens eine cDNA-Bibliothek von einer Gewebeveränderung, wie sie hierin beschrieben ist, oder eines Teils davon angelegt, die/der eine Aktivierung eines Gens der HMGI(Y)-Familie oder eines Derivates aufweist, so kann die Aktivierung durch eine entsprechende Chromosomenaberration kenntlich sein.

Bei den erfindungsgemäßen Verfahren wird das Screenen typischerweise unter Bedingungen niedriger Stringenz vorgenommen. Unter Bedingungen niedriger Stringenz soll dabei verstanden werden, daß durch Herabsetzen bspw. der Hybridisierungstemperatur oder Modifikation der Waschbedingungen (z.B. Erhöhung der Salzkonzentration) auch eine Bindung an solche Nukleinsäuren erfolgt, deren Homologie zu der Sequenz der Sonde deutlich reduziert ist, wobei zunächst ggf. auch eine Erfassung falsch-positiver Nukleinsäuresequenzen in Kauf genommen wird, da eine letztendliche Klärung ob es sich um ein positives Signal bzw. Ergebnis handelt, durch Sequenzierung und Sequenzanalyse der identifizierten Sequenzen erfolgt bzw. erfolgen kann und somit eine Ausscheidung der falsch-positiven Sequenzen zuläßt.

Bei den erfindungsgemäßen Verfahren kann, nachdem durch einen Vergleich des RNA - oder cDNA - Musters von transfizierten Zellkulturen mit dem von Kontrollkulturen, oder durch ein positives Signal in einem PCR-Test unter Verwendung von Primer(paaren), die Sequenzen viraler Nukleinsäuren entsprechen, oder durch ein positives Signal beim Screenen einer cDNA-Bibliothek mit einer virusspezifischen Sonde, oder durch Analyse der cDNA-Klone auf virale Sequenzen oder Vergleich mit einer cDNA-Bibliothek aus normalem mesenchymalen bzw. ggf. epithelialen Gewebe, festgestellt wurde, daß virale Elemente in den hierin beschriebenen Gewebeveränderungen bzw. den davon abgeleiteten Zellkulturen vorhanden sind, weiterhin vorgesehen sein, daß die viralen Elemente identifiziert und/oder klassifiziert und ausschließlich für die Impfstoffherstellung verwendet werden.

Die erfindungsgemäßen Vorrichtung zur Bestimmung eines an der Pathogenese beteiligten Virus umfaßt ein Genprodukt von Genen der HMGI(Y)-Familie, das an einen Träger gebunden ist. Im Zusammenhang mit der erfindungsgemäßen Vorrichtung sowie deren möglichen Verwendungen umfaßt der Begriff Genprodukt auch Teile der Genprodukte oder Derivate der Genprodukte. Dabei liegt der Definition von Genprodukten auch die oben gegebene Definition von Genen der HMGI(Y)-Familie zugrunde.

Das Genprodukt ist dabei an ein Trägermaterial gekoppelt, das vom Fachmann auf dem Gebiet entsprechend ausgewählt wird. Als Trägermaterialien eignen sich all jene Materialien, die eine Bindung von Proteinen oder Derivaten, sei es direkt oder indirekt erlauben. Geeignete Trägermaterialien finden sich typischerweise im Bereich der Chromatographiematerialien. Eine derartige Bindung kann auch eine Adsorption sein bzw. eine reversible Bindung. Da es sich bei den Genprodukten der HMGI(Y)-Familie um Proteine handelt, können die den Fachleuten auf dem Gebiet bekannten Verfahren und Verbindungen zur Immobilisierung von Proteinen verwendet werden.

Hinsichtlich der Gestaltung des/der an das Trägermaterial gebundenen Genproduktes bzw. Genprodukte ist im wesentlichen darauf zu achten, daß derjenige Bereich an das Trägermaterial gebunden ist bzw. für Bindung viraler Nukleinsäure zur Verfügung steht, der für das Binden an die virale Nukleinsäure verantwortlich ist. Insoweit kann das entsprechende Genpro-

Die Erfindung wird anhand der folgenden Beispiele, experimentellen Befunde und Figuren weiter erläutert. Dabei zeigt
- Fig. 1: die Größenverteilung der Myome mit normalem Karyotyp (graue Säulen) sowie mit 12q14-15-Aberrationen (schwarze Säulen);
- Fig. 2: eine Übersicht der in Beispiel 4 verwendeten Vektoren;
- Fig. 3a-c: einen Sequenzvergleich verschiedener Sequenzen aus Myomgewebe mit adenoviralen Sequenzen;
- Fig: 4a-b eine: vergleichende Analyse verschiedener aus Myomgewebe erhaltener Sequenzen;
- Fig. 5: Mögliche HMGI(Y)-Bindungsstellen in der Promotorsequenz des adenoviralen Proteins EIA; und
- Fig. 6: das Ergebnis einer PCR-Analyse, mit der die Anwesenheit eines adenoviralen DNA-Fragmentes in verschiedenen Gewebeveränderungen untersucht wurde.

### Beispiel 1

Die Ergebnisse bisheriger zytogenetischer Studien an Uterus-Myomen sind widersprüchlich bezüglich möglicher Korrelationen zwischen der Tumorgröße und dem Auftreten klonaler Chromosomenaberrationen. Das Problem dieser Studien ist allerdings, daß die untersuchten Tumoren möglicherweise z. B. nach Größe selektiert sind. Da die Frage einer möglichen Korrelation aber auch damit zusammen hängt, ob die Chromosomenaberrationen sekundär auftreten und das Wachstumspotential der betroffenen Tumoren verstärken, wurde eine Studie an unselektierten Myomen durchgeführt. Untersucht wurden dabei nur Myome nach Hysterektomie, wobei alle Tumoren untersucht wurden, die durch Tastuntersuchung des operativ entfemten Uterus nachweisbar waren.

Insgesamt wurden 155 Myome von 96 Patientinnen zytogenetisch untersucht. Dabei zeigten 28% der Myome klonale Karyotypänderungen. Auf die drei Hauptkaryotypengruppen mit normalem Karyotyp, Aberrationen der Chromosomenregion 12q14-15 und Deletion des langen Arms von Chromosom 7 entfielen 72%, 12% und 8%. Die durchschnittliche Tumorgröße für die Gruppen ist Tabelle 3 zu entnehmen.

**Tab. 3: Durchschnittliche Myom-Größe in den drei Hauptkaryotyp-Gruppen mit 12q14-15-Aberrationen, Deletionen des langen Arms von Chromosomen 7 und normalem Karyotyp**

| | |
|---|---|
| Karyotyp-Gruppe | durchschnittliche Größe [cm] ± Standardabweichung[cm] |
| normaler Karyotyp | 3,4 ±2,1 |
| 12q14-15 Aberrationen | 8,9 ±5,6 |
| Deletion des Chromosoms 7 | 3,5 ± 2,0 |

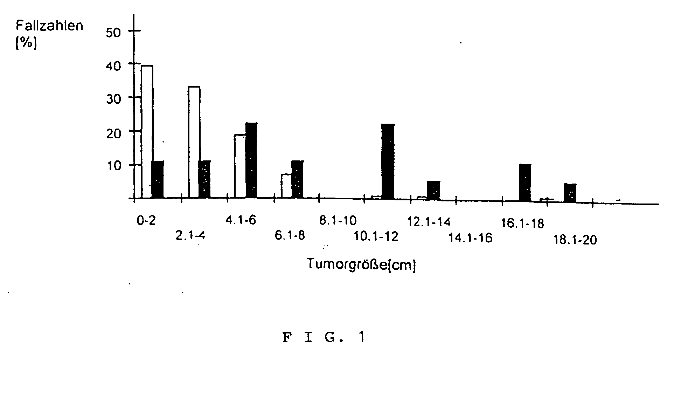

Die Ergebnisse zeigen deutlich, daß das Auftreten von 12q14-15-Aberrationen, die molekulargenetisch mit Mutationen im oder im Bereich des HMGIC-Gens korrelieren, mit einer hochsignifikanten Größenzunahme der entsprechenden Myome einhergehen, wenn man sie entweder mit Myomen mit normalem Karyotyp oder mit Deletionen am langen Arm von Chromosom 7 vergleicht. Die Unterschiede werden nicht nur beim Vergleich der Mittelwerte, sondern auch der Verteilungen der Tumorgrößen der einzelnen Tumorgruppen, wie dargestellt in Fig.1, deutlich.

Zusammengefaßt führt offenkundig das Auftreten von Chromosomenaberrationen der Chromosomenregion 12q14-15, das mit der verstärkten Expression von HMGIC-Gen oder der Expression veränderter Transkripte dieses Gens einhergeht, zu einem verstärkten Tumorwachstum.

### Beispiel 2

Mutationen im Bereich des HMGIC- oder HMGIY-Gens manifestieren sich zytogenetisch durch Chromosomenaberrationen der Region 12q14-15 bzw. 6p21. Zumindest theoretisch ist aber durchaus denkbar, daß die zytogenetisch erkennbaren Aberrationen nur "die Spitze des Eisberges" darstellen und ein wesentlich größerer Teil der Mutationen der beiden genannten Gene mit zytogenetisch nicht darstellbaren chromosomalen Umbauten einhergeht. Wäre dies der Fall, könnte es für eine Schlüsselrolle der genannten Aberrationen bei der Tumorentwicklung insgesamt im Sinne einer primären Mutation sprechen. Eine Methode zum Nachweis der "versteckten" Umlagerungen ist die Fluoreszenz-in-situ-Hybridisierung (FISH). Es wurde an einer Serie von 40 Myomen mit anscheinend normalem Karyotyp FISH-Experimente durchgeführt, für die Cosmid- und PAC-Sonden eingesetzt wurden, die den Lokalisierungsort von HMGIC- bzw. HMGIY-Gen abdecken. Die Sonden wurden dabei so gewählt, daß ein Bereich von ca. 150 kb 5' bis 40 kb 3' vom HMGIC-Gen und von 30 kb 5' bis 40 kb 3' vom HMGIY-Gen abgedeckt wurde. Alle Myome wurden mit den Sonden für beide Gene untersucht; analysiert wurden jeweils mindestens 20 Metaphasen. In keinem Fall ergaben sich Hinweise auf mit Mitteln der konventionellen Zytogenetik nicht erkennbare, verdeckte chromosomale Umlagerungen der untersuchten Bereiche.

Berücksichtigt man die Häufigkeit der Myome ohne erkennbare zytogenetische Veränderungen (s. Beispiel 1) und die Ergebnisse der in diesem Beispiel dargestellten Untersuchungen, ist es wahrscheinlich, daß die Mutationen der Gene der HMGI(Y)-Familie bei der Mehrzahl der Uterus-Myome keine Schlüsselrolle spielt.

### Beispiel 3

Unabhängig davon, ob diese Veränderungen primär oder sekundär sind, wird als molekulargenetische Basis der 12q14-15- und 6p21-Aberrationen bei Uterus-Myomen angenommen, daß es durch die chromosomalen Umlagerungen zu einer Expression/verstärkten Expression oder einer Expression aberranter Transkripte des HMGIC-Gens bzw. HGMIY-Gens kommt, die in normalem Uterus-Gewebe fehlt. Für das HMGIC-Gen ist mittels einer RT-PCR in normalem Uterus-Gewebe im Regelfall keine HMGIC-Genexpression nachweisbar. Es wurden mit dieser Methode 40 Myomgewebe mit anscheinend normalem Karyotyp untersucht. Ziel der Untersuchung war wiederum festzustellen, ob bei diesen Myomen, wie bei solchen mit 12q14-15-Veränderungen, Hinweise auf eine HMGIC-Genexpression zu finden seien.

Bei keinem dieser Myome waren Hinweise auf eine Expression zu finden, so daß auch aus diesen Ergebnissen darauf geschlossen werden kann, daß bei der Entstehung dieser Myome HMGIC-Genexpression nicht das primäre Ereignis ist.

### Beispiel 4

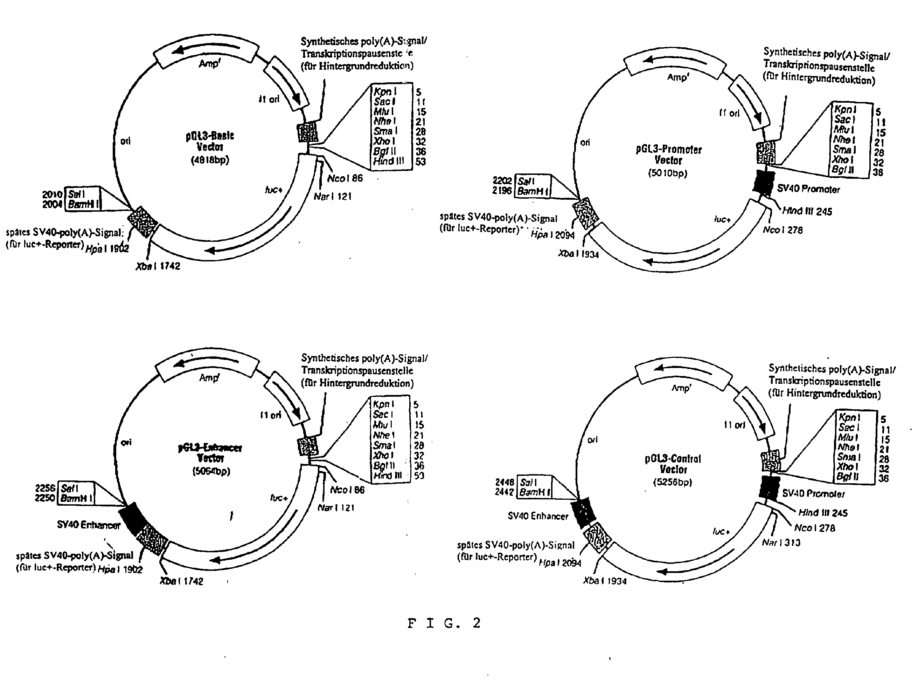

Für die Untersuchung der Wirkung von HMGIC auf den SV 40-Promotor werden zwei Vektorsysteme in eine Zelle transfektiert. Dies sind der Expressionsvektor H₃Hₓ für HMGIC und der "pGL3 Luciferase Reporter Vektor" der Firma Promega. Das komplette "pGL3 Luciferase Reporter Vektor"-System von Promega umfaßt 4 verschiedene Vektoren, die es ermöglichen, DNA-Abschnitte hinsichtlich Promotor- oder Enhancer-Regionen zu untersuchen. Diese Vektoren sind in Fig. 2 dargestellt. Für die Untersuchung von Promotor-Abschnitten wird der Vektor "pGL3-Enhancer" benötigt. Der Vektor "pGL3-Promotor" ist für die Untersuchung von Enhancer-Elementen vorgesehen. Desweiteren wird der Vektor "pGL3-Promotor" bei diesem Versuch verwendet, um die Wirkungsweise von HMGIC auf einen SV40-Promotor bzw. Promotoren anderer Polyomaviren zu testen. Hierzu wird der Vektor H₃Hₓ mit dem Vektor "pGL3-Promotor" kotransfektiert.

Der Vektor "pGL3-Control" dient als Positivkontrolle für das System, eine Transfektion ausschließlich mit dem Vektor "pGL3-Promotor" als Negativkontrolle.

Die einzelnen Vektoren haben abgesehen von Promotor- und Enhancer-Elementen die gleiche Grundstruktur. Sie verfügen über eine modifizierte Kodierungsregion für Feuerfliegen-Luciferase *(Photinus pyralis) (luc+*), welche für die Untersuchung der Transkriptionsaktivität in transfektierten eukaryotischen Zellen gewählt wurde. Desweiteren enthalten sie einen prokaryotischen Replikationsursprung zur Vermehrung in *E.coli,* ein Ampicillin-Resistenzgen für die Selektion, einen Replikationsursprung von filamentösen Phagen (f1 ori) für die Produktion einzelsträngiger DNA (ssDNA) und eine "multi cloning site" (MCS) 3' und 5' des Luciferase-Gens.

Der "pGL3-Promotor"-Vektor ist 5010 Basenpaare groß und enthält im Gegensatz zum "pGL3-Enhancer" einen SV40-Promotor und keinen Enhancer. DNA-Fragmente, die vermeintliche Enhancer-Sequenzen enthalten, können 3' oder 5' des Luciferase-Gens eingesetzt werden und so zu einer Verstärkung führen. Desweiteren kann der SV40-Promotor durch andere Polyomavirus-Promotoren ersetzt werden.

Der Vektor "pGL3-Control" (5256 Basenpaare) enthält einen SV40 Promotor und eine Enhancer-Sequenz, was in den meisten Säugerzellen zu einer starken Expression von *luc+* führt. Dieser Vektor dient zur Kontrolle der Transfektionseffizienz und setzt den internen Standard für die Promotor- und Enhancer-Aktivität der anderen Vektoren.

Für die Untersuchung haben sich HeLa-Zellen als geeignet erwiesen, da sie sehr leicht zu handhaben sind, den Vorgang der Transfektion nahezu ohne Schaden überstehen und keine Expression von HMGIC aufweisen (das Nichtvorhandensein der HMGIC Expression wurde durch "Northern Blot" nachgewiesen). Die Zellen werden für die Transfektion in Platten mit 6 Näpfen herangezogen.

Für den Vorgang der Transfektion wurden 2µg DNA mit Zellmedium (TC 199), ohne Kälberserum und Antibiotika, gemischt (beides kann die Komplexbildung stören), Endvolumen 100µl.

Zu dem DNA-Gemisch werden 10µl "SuperFect" zugegeben. Nach dem Mischen inkubiert man 10 min bei Raumtemperatur, wobei sich Komplexe aus der DNA und dem "SuperFect" bilden.

Während der Inkubation wird von den Zellen das alte Medium abgesaugt und die Zellen mit 1x PBS gespült. Das Gemisch aus "SuperFect" und DNA wird mit 800µl Zellmedium mit 20% Kälberserum gemischt, welches anschließend auf die Zellen gegeben wird. Nach einer Inkubation (im Brutschrank bei 37°C und 6% CO₂) von 16-18 Stunden wird frisches Medium (20%) zugegeben und weitere 8-32 Stunden inkubiert.

Die Auswertung des Versuches erfolgt mit dem "Luciferase Assay Kit" von Stratagene (s.u.).

### Luciferase-Extraktion und Bestimmung der Luciferase-Konzentration:

Nach der Inkubation der transfektierten Zellen wird das Medium abgesaugt und 500µl 1x Zelllysepuffer zugegeben. Nach 15-minütiger Inkubation, bei Raumtemperatur auf einem Schüttler, lysieren die Zellen. Das Zelllysat wird in Eppendorf-Cups überführt. Dieses ist für kurze Zeit bei 4°C lagerbar. Über längere Zeit kann es bei -80°C gelagert werden, hierbei geht jedoch bis zu 50% der Luciferase-Aktivität verloren.

Für die Bestimmung der Luciferasekonzentration werden 20µl Zellysat mit 100µl "Luciferase assay reagent" (LSA) (beides sollte Raumtemperatur haben) gemischt. Das Luciferin im Reaktionsgemisch wird unter ATP Verbrauch umgesetzt, wobei Lichtquanten entstehen.

Luciferase-Substrat (Luciferin) + ATP + O₂ → Licht (560 nm) + Oxyluciferin + AMP + PPᵢ

Die emittierten Lichtquanten können mit einer Photozelle eines Luminometers gemessen werden. Die ermittelten Werte werden in relativen Lichteinheiten (relative light units (RLU)) angegeben und vermitteln im Verhältnis zu anderen Werten Auskunft über die gebildete Menge an Luciferase.

### Ergebnisse:

Bisher wurden zwei voneinander getrennte Meßreihen durchgeführt. Weitere Messungen, vor allen Dingen mit Promotorregionen der BK- und JCV-Viren können leicht vorgenommen werden, im vorliegenden Testsystem durch Klonierung der entsprechenden Promotorregionen in die Testvektoren.

Die Ergebnisse der ersten 2 Meßreihen sind in Tabelle 4 zusammengefaßt.

**Tabelle 4: Ergebnisse der Transfektionsversuche**

| | Positivkontrolle | Negativkontrolle | 1µg H₃Hₓ (1µg pGL3-P) | 0,5.µg H₃Hₓ (1µg pGL3-P) | 0,25 µg H₃Hₓ (1µg pGL3-P) |
|---|---|---|---|---|---|
| relative Lichteinheiten 1. Versuch | 14.500 | 1.800 | 4.300 | 8.800 | 3.800 |
| relative Lichteinheiten 2. Versuch | 15.100 | 2.100 | 5.100 | 9.400 | 4.800 |

Die Meßergebnisse liegen über denen der Negativkontrolle und unter denen der Positivkontrolle mit sehr starkem Promotor, was deutlich eine leichte Regulierung der virale Promotorregion durch HMGIC belegt.

Dies belegt die vorstehend beschriebene Beteiligung von Viren an der Entstehung von Leiomyomen, Endometriumpolypen und Endometriose.

### Beispiel 5

In diesem Beispiel werden die Ergebnisse eines PCR-Tests dargestellt, der durchgeführt wurde, um nach Adenovirus-spezifischen DNA-Sequenzen in Myomgeweben zu suchen. Dabei stehen für alle 6 Subgenera der Adenoviren spezifische Oligonukleotide zur Amplifikation von viralen DNA-Sequenzen zur Verfügung.

Unter Anwendung des PureGene Kits (Fa. Gentra, deutscher Vertrieb Biozym) wurde DNA aus 16 Myomen, 6 Zellkulturen von Myomen, 1 Myometrium und 2 Blutproben isoliert.

DNAs von 8 Myomen, allen 6 Zellkulturen, des Myometriums und der Blutproben wurden in eine PCR eingesetzt. Verwendet wurden die Oligonukleotidpaare HsgA1 (SEQ: ID. No 1: aaggtgtcaatyatgtttg) /HsgA2 (SEQ ID.NO. 2: acggttacttkttt) und HsgB1 (SEQ ID No. 3: tctattccctacctggat) /HsgB2 (SEQ ID. No. 4: actcttaacggcagtag) aus der Sequenz des Hexon-Gens, die jeweils Adenovirus-DNA der Gruppe A bzw. B amplifizieren ( Pring-Akerblom et al., J. Med. Virol. 58, 87-92, 99). Das Oligonucleotidpaar HsgA amplifiziert ein Fragment von 299 bp, das Oligonucleotidpaar HsgB ein Fragment von 465 bp. Als Positivkontrollen wurden Virus-DNA Proben der Adenovirus Subgenera A (Ad18) und B (Ad7) verwendet, die von Frau Dr. Patricia Pring-Akerblom, Medizinische Hochschule Hannover, Institut für Virologie und Seuchenhygiene, 30623 Hannover zur Verfügung gestellt wurden. Folgender PCR-Ansatz wurde verwendet:
500 ng DNA (Myome/Blut) bzw. 50 ng Virus-DNA
1.5 mM MgCl₂
je 0.5 µM Primer
5 µl 10xPCR-Puffer ohne MgCl₂ (Sigma)
200 µM dNTP
2.5 U Taq-Polymerase (Sigma)

Jeder Ansatz enthielt 50 µl Gesamtvolumen. Folgende Zyklen wurden durchgeführt:

| | | |
|---|---|---|
| 1 × | 6 min | 95°C |
| 40× | 40 sec | 92°C |
| | 30 sec | 41°C |
| | 40 sec | 72 °C |
| 1 × | 5 min | 72°C |

Der gesamte Ansatz wurde in einem 1-%igen Agarosegel aufgetragen.

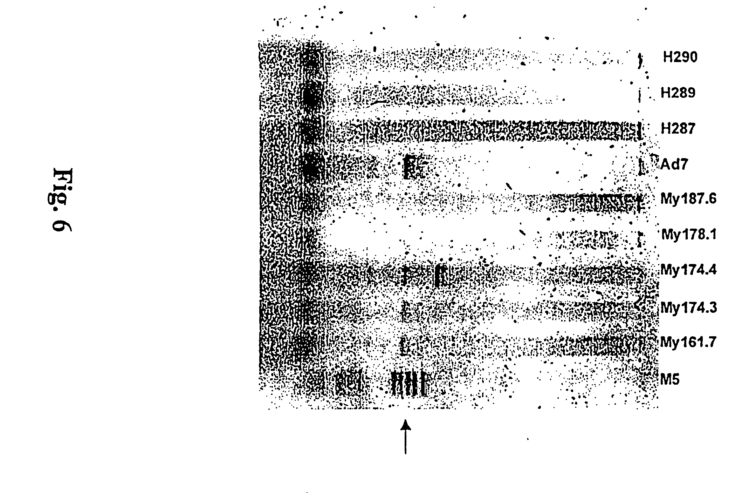

Fig. 6 zeigt das Ergebnis der PCR-Analyse zur Prüfung auf mögliche DNA-Sequenzen von Adenoviren mit Konsensus-Primern der Gruppe B. Mit Ausnahme der Virus-Kontroll-DNA konnte mit Primerpaar HsgA keine Amplifikation des 299 bp-Fragmentes nachgewiesen werden. Mit Primerpaar HsgB wurde bei vier DNA-Fragmenten von Uterus-Leiomyomen (My178.1; My174.3; My174.4; My161.7) (Zellkultur und primäres Tumorgewebe) und der viralen Kontroll-DNA (humanes Adenovirus 7) eine Amplifikation des 465 bp-Fragmentes nachgewiesen. Weder aus Blut-DNA noch aus DNA aus normalem Myometrium aus einem Uterus myomatosus (My187.6) oder den untersuchten Lungen-Hamartomen (H) wurde ein entsprechendes Produkt amplifiziert. M5 bezeichnet einen Marker (DNA-Standard V, Roche, Penzberg), Pfeil: Lage des spezifischen 465bp-Fragmentes. Damit konnte eindeutig der Nachweis viraler DNA-Sequenzen des Adenovirus-Typ B in den Myomgeweben erbracht werden.

### Beispiel 6

Zur näheren Charakterisierung der PCR-Produkte wurden diese in einen geeigneten Vektor kloniert und aus dem Vektor von beiden Seiten sequenziert. Hierzu wurde ausgehend von den Ergebnissen der PCR-Analysen (siehe Beispiel 5) die dem 465 bp-Amplifikat entsprechende DNA (6 Fragmente aus 6 Myom-DNA-Proben und 1 Fragment aus der Virus-Kontroll-DNA Ad7) mit Hilfe des QIAEX II-Kits (Qiagen, Hilden, Deutschland) nach Herstellerangaben (QIAX II Handbook Ausgabe August 1996, S. 12-13) aus dem Agarose-Gel eluiert. Ferner wurde ein größerers Fragment, das aus der DNA My174.3 amplifiziert wurde (Vergl. Bsp. 5), eluiert. Im folgenden Schritt wurde die gereinigte DNA in den Vektor pGEM-T Easy (Promega, Madison, USA) nach Herstellerangaben (Technical Manual pGEM-T and pGEM-T Easy Vector Systems, S. 11) ligiert und das Vektorkonstrukt in *E.coli* kloniert (Technical Manual pGEM-T and pGEM-T Easy Vector Systems, S. 12-13). Plasmid-DNA positiver Bakterienklone wurde mit Hilfe des QIAprep-Kits (Qiagen, Hilden, Deutschland) nach Herstellerangaben (QIAprep Miniprep Handbook Ausgabe April 1998, S. 18-19) isoliert. Die Sequenzierung der klonierten Inserts erfolgte unter Verwendung der Oligonukleotide M 13 universal und M 13 revers und mit Hilfe einer automatischen Sequenziereinheit (373 Applied Biosystems, Weiterstadt, Deutschland). Die vergleichende Analyse der Sequenzen erfolgte mit Hilfe der vom amerikanischen National Center for Biotechnology (NCBI) Information des National Institute of Health per Internet veröffentlichten Datenbanken (Zugang: http://www.ncbi.nlm.nih.gov/) und Suchmethoden (Advanced BLAST, Datenbank "nr" ohne Angabe einer bestimmten Spezies).

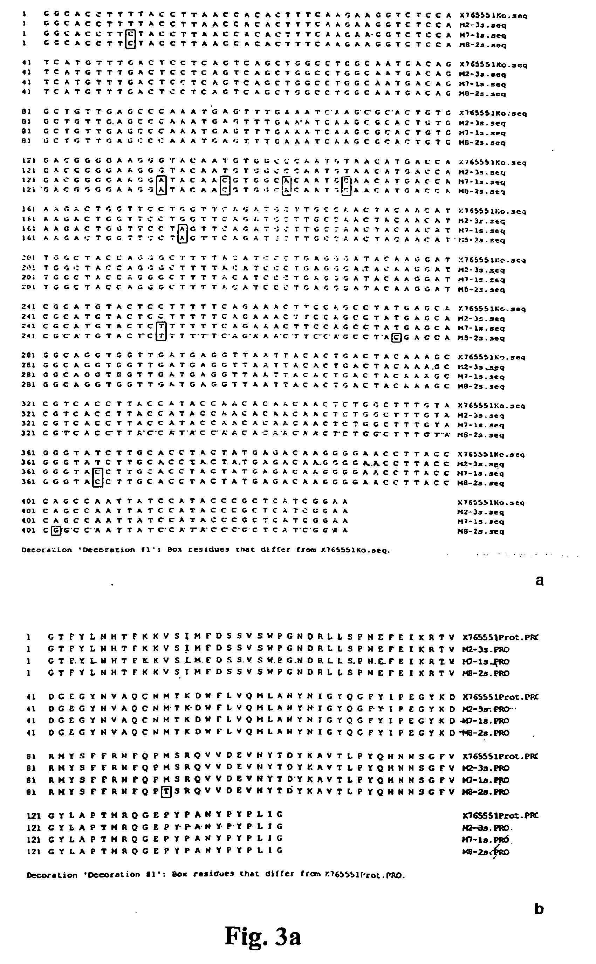

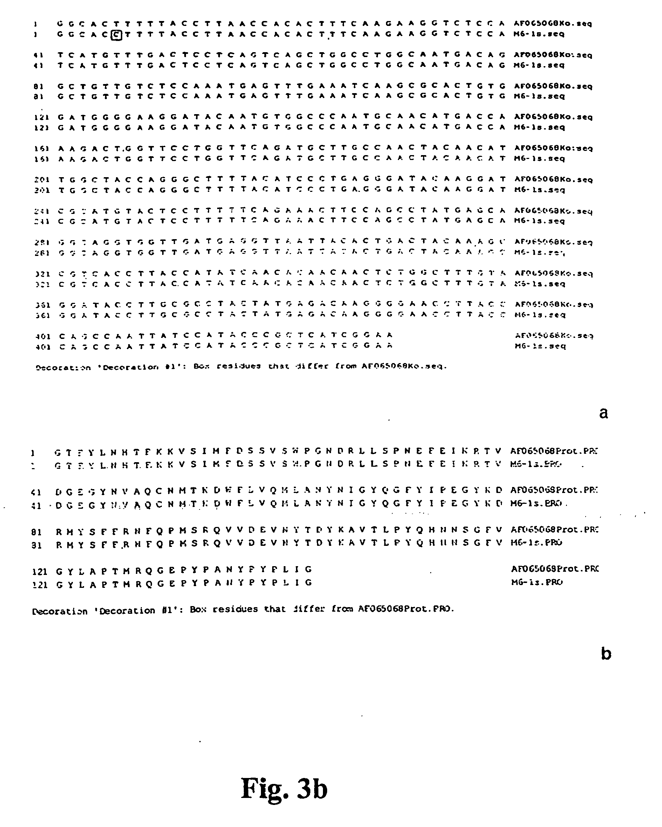

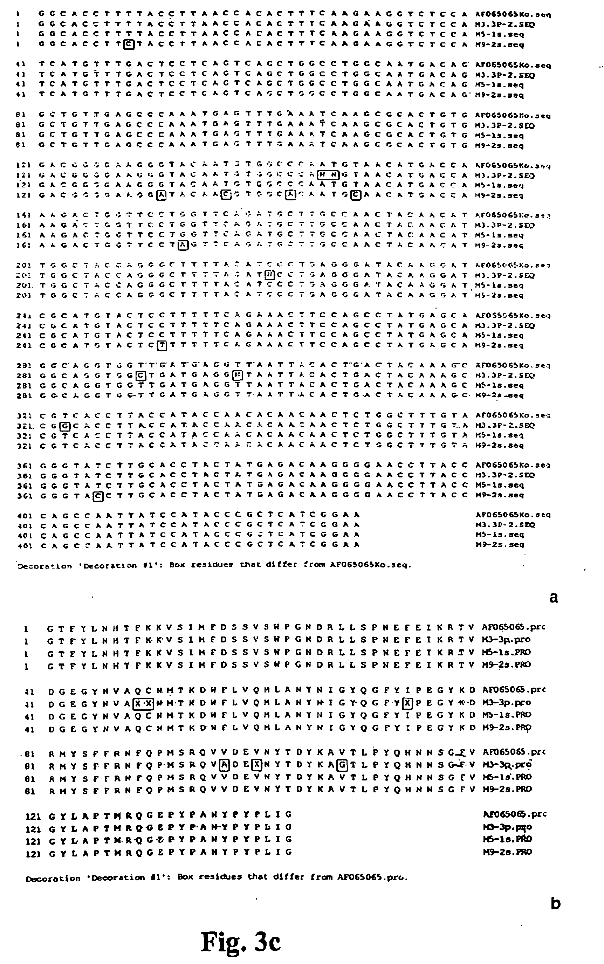

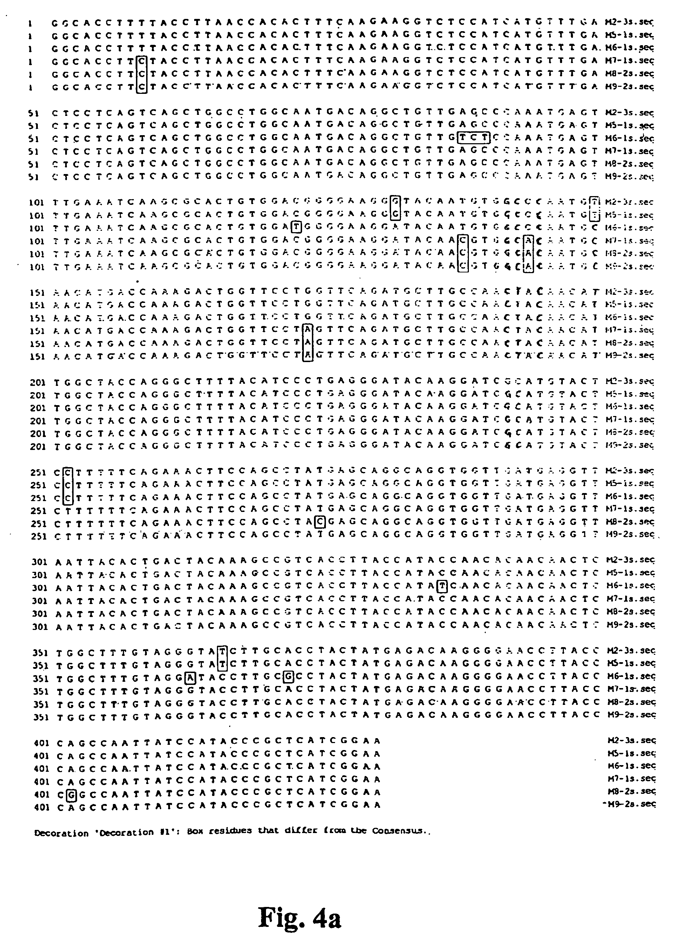

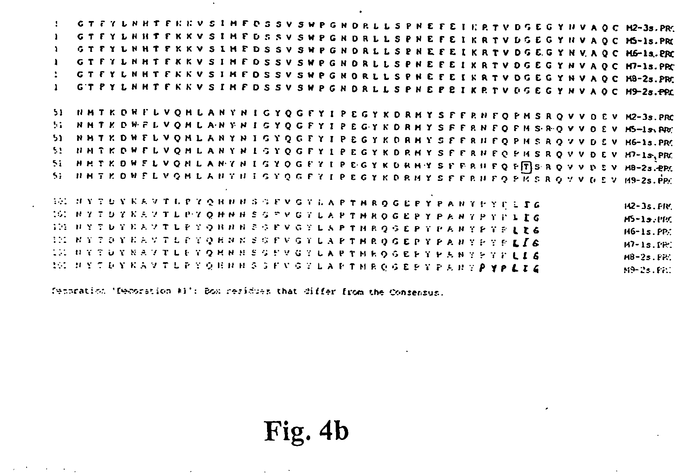

Ergebnisse: Die amplifizierten Sequenzen aus allen Tumoren und Tumorzellkulturen entsprechen (mit einigen Abweichungen, siehe Abb. 3, Abb. 4 und Tabelle 5) dem 465 bp langen PCR-Fragment der Positivkontrolle. Die Sequenz, die bei der Analyse des größeren Fragment des Amplifikats aus My174.4 erhalten wurde, ergab in der vergleichenden Analyse keinen Match zu einer viralen Sequenz.

**Tabelle 5**

| PCR - Produkt | Bester Match Zugangsnummer | Anzahl der Mutationen | Besonderheiten |
|---|---|---|---|
| M 3-3 (Kontrolle AD7) | AF065065 | 2 | 4xN, 2 Austauschmutationen |
| M 2-3 (Myom-DNA) | X765551 | 0 | |
| M 5-1(Myom-DNA) | AF 065065 | 0 | |
| M 6-1 (Myom-DNA) | AF 065068 | 1 | Keine Austauschmutation |
| M 7-1 (Myom-DNA) | X765551 | 8 | Keine Austauschmutation |
| M 8-2 (Myom-DNA) | X765551 | 10 | 1 Austauschmutation |
| M 9-2 (Myom-DNA) | AF065065 | 8 | Keine Austauschmutation |

Damit wurde gezeigt, daß die viralen Sequenzen, die aus den einzelnen Myomen amplifiziert wurden, der Sequenz des Adenovirus Typ 7 am ähnlichsten sind und untereinander an Hand von Punktmutationen unterschieden werden können.

### Erläuterungen zu Fig. 3a - c, Fig. 4a-b und Tabelle 5

Die vergleichende Analyse der DNA- und Proteinsequenzen ergab den Nachweis, einer hohen Homologie der aus den Myomengeweben amplifizierten Sequenzen zu veröffentlichten Sequenzen aus der Hexonregion des Adenovirus Typ 7. Die einzelnen DNA-Sequenzen weisen Punktmutationen auf und unterscheiden sich untereinander. Mit Ausnahme einer Punktmutation, die zu einem Aminosäureaustausch in der Proteinsequenz von M 8-2 führt, erweisen sich alle übrigen Mutationen als sogenannte stille Mutationen. Die Sequenzunterschiede sind jeweils in den Abbildungen Fig. 3a - c durch Kästchen markiert. Verwendete Nomenklatur: X765551, AF 065068, AF 65065: veröffentlichte Sequenzen adenoviraler Hexongene, M 3-3P: Positivkontrolle (siehe Beispiel 5), M 2-3, M 5-1, M 6-1, M7-1, M 8-2, M 9-2: verschiedene Sequenzen von Amplifikaten aus Myomgewebe.

Die Abbildung Fig. 4a - b zeigt eine vergleichende Analyse aller aus den verschiedenen Myomgeweben erhaltenen DNA-Sequenzen. Die Unterscheidungen sind durch Kästchen markiert. Die verwendete Nomenklatur entspricht derjenigen von Fig. 3 a-c

### Beispiel 7

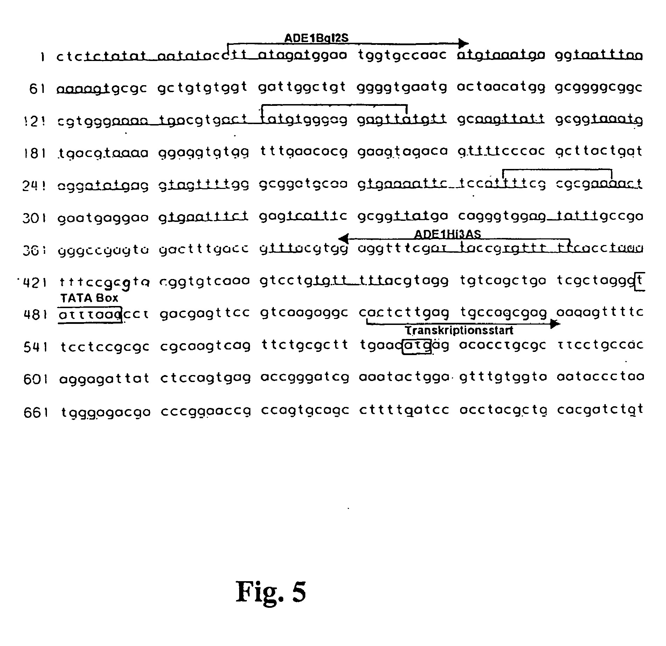

HMGI(Y)-Proteine können durch die Bindung an virale Promotoren die Expression von viralen Proteinen beeinflussen. Anhand der veröffentlichten Sequenz des Promotors des Gens E1a (Zugangsnummer X03000 oder NCBI-Datenbank; E1a ist ein adenovirales Gen, dessen Genprodukt eine transformierende Funktion zugeordnet wird) und mittels veröffentlichter Daten zur Bindungsmodalität von HMGI(Y) an DNA -Sequenzen (vgl. Yic et al., Molecular and Cellular Biology 17: 3649 - 3662, 1997) wurde überprüft, ob HMGI(Y) an die Promotorsequenz binden kann. Proteine der HMGI-Y Gruppe enthalten drei Bindungsdomainen, von denen jeweils 2 parallel an DNA-Sequenzen, die aus einer Reihung von mindestens 4 Adeninen und Thymidinen bestehen, binden können. Diese DNA-Sequenzen liegen idealerweise 10 oder 20 Basenpaare von einander entfernt, da die bindenden HMGI-Y Proteine auf Grund der Lage der Bindungsdomainen 1-2 helikale Windungen der DNA umspannen können (Yie et al., Molecular and Cellular Biology, 17: 3649-3662, 1997). Binden HMGI-Y Proteine auf die beschriebene Weise an zelluläre oder virale Promotoren, so wird die Promoter-vermittelte Wirkung im Sinne einer Aktivierung oder Hemmung modifiziert. Mittels Sequenzvergleich unter Verwendung der über NCBI veröffentlichten Datenbanken (siehe Beispiel 6) wurde die Promotersequenz des adenoviralen Proteins E1A identifiziert (Zugangsnummer X03000, Nukleotide 1-511, Quelle: AD7). An Hand der beschriebenen Kriterien wurden zahlreiche Bindungsstellen für HMGIY Proteine ermittelt (Vergl. Fig. 5). Exemplarisch sind zwei dieser identifizierten Bindungsstellen, die parallel durch zwei Bindungsdomainen eines HMGI-Y Proteins gebunden werden können, durch eine Klammer verbunden worden.

Hiermit wurde gezeigt, daß HMGI-Y Proteine an die Promotersequenz binden können.

### Beispiel 8

Es wurden anhand der über NCBI publizierten Sequenz des linken Endes von AD7 (Zugangsnummer X 03000) die Oligonucleotide ADE1Bg12S: (SEQ ID. NO. 5): gaa gat ctt tat aga tgg aat ggt gcc aac at und ADE1Hi3AS (SEQ. ID. NO. 6): ccc aag ctt aaa act ctt ctc gct ggc agt c ausgewählt, die an die Promotorsequenz des E1A-Gens des Adenovirus 7 binden können. Das Oligonucleotid ADE1Bg12S enthält eine Bg1 II-Schnittstelle und das Oligonucleotid ADE-Hi3AS enthält eine HindIII-Schnittstelle. Beide Schnittstellen sind in der oben angegebenen Sequenz unterstrichen. Unter der Verwendung der Oligonukleotide wurde ein 521bp langes Fragment aus der AD7-Promoter-Region amplifiziert und über die Schnittstellen Bgl1 und HindIII mittels Standardmethoden (Maniatis) in den Luciferase-Reportervektor pGL3-Enhancer (Promega) kloniert (pAD7PROM). Als Template (Matrize) wurde die AD7-DNA, die bereits im Beispiel 5 als Positivkontrolle gedient hat, verwendet. Das amplifizierte Fragment fungiert dann als Promoter für das im Vektor vorhandene Firefly-Luciferase-Gen, dessen Aktivität durch ein Luciferase-Assay (Dual-Luciferase-Reporter-Assay-System, Promega) meßbar ist. Die Co-Transfektion des o.g. Konstruktes mit einem HMGIC-Expressionsvektor (H3HX) sowohl in Hela-Zellen als auch in primäre Myometrium-Zellen aus der 1. Passage der Gewebekultur erbrachte den Nachweis einer Modifizierung der E1A-Promotorfunktion durch HMGIC. Alle Transfektionen wurden mit dem Superfect nach dem Protokoll der Firma Qiagen durchgeführt. In Abänderung zum Original-Protokoll wurde jeweils 1 µg der jeweiligen Konstrukte verwendet und die Zellen wurden nicht mit PBS gewaschen. Die Inkubation wurde von wenigen Stunden auf eine Über-Nacht-Inkubation verlängert und nach dieser wurde das Superfekt nicht abgenommen, sondern 3 mL Medium zu den Kulturen gegeben. Damit wurde gezeigt, daß die Expression des transformierenden adenoviralen Proteins E1A durch die Bindung von HMGIC-Proteinen in der viralen Promoter-Region beeinflußt wird. Als Negativ-Kontrollen wurden zwei weitere Co-Transfektionen durchgeführt, wobei einmal der Expressionsvektor keine klonierte *HMGIC*-Sequenz enthielt und zusätzlich wurde transfektiert ohne Zugabe des *HMGIC*-Expressionkonstrukts. Als Positiv-Kontrolle diente der pGL3-Kontroll-Vektor, der einen SV40-Promoter und SV40-Enhancer enthält. Um Ungenauigkeiten bei der Zellkultur auszuschließen, wie z.B. unterschiedliche Anzahl der Zellen pro Zellkulturschale, unterschiedliche Effizienz bei Transfektion und Zell-Lysis, wird die Aktivität des experimentellen Reporterkonstrukts (s.o.) durch Co-Transfektion mit einem internen pRL-Kontroll-Vektor (pRL-TK, Renilla-Luciferase) normalisiert. Die Durchführung der Luciferase-Messungen erfolgte nach dem Protokoll des "Dual-Luciferase-Reporter-Assay-System" von Promega.

### Beispiel 9

Ein weiteres Beispiel für die Induktion von Gewebeveränderungen durch die Infektion der Gewebezellen mit Adenoviren sind die Lungenhamartome. Dieses Beispiel beschreibt die Strategie zur Überprüfung und zum Nachweis von adenoviralen Genomen in verschiedenen Lungenhamartomgeweben.

Unter Anwendung des PureGene Kits (Fa. Gentra, deutscher Vertrieb Biozym) wurde DNA aus 10 Zellkulturen von Hamartomen isoliert.

DNAs von 7 Zellkulturen wurden in eine PCR eingesetzt. Verwendet wurden die Oligonukleotidpaare HsgA1/HsgA2, HsgB1/HsgB2, HsgC1 [(SEQ. ID. NO. 7): acctttgactcttctgt)]/HsgC2 [(SEQ. ID.NO 8): tccttgtatttagtatc], HsgD1 [(SEQ.ID.NO. 9): ccatcatgttcgactcct]/HsgD2 [(SEQ: ID.No. 10): aggtagccggtgaagcc], HsgE1 [(SEQ: ID: NO.11): gactcttccgtcagctgg]/HsgE2 [(SEQ: ID: NO. 12): gctggtaacggcgctct] und HsgF1 [(SEQ. ID. No. 13): atttctattccttcgcg] /HsgF2 [(SEQ: ID. No.14): tcaggcttggtacggcc], aus der Sequenz des Hexon-Gens, die jeweils Adenovirus-DNA der Gruppen A bis F amplifizieren (Pring-Akerblom et al., J. Med. Virol. 58, 87-92, 99). Oligonukleotidpaar HsgA amplifiziert ein Fragment von 299 bp, Oligonukleotidpaar HsgC von 269 bp, Oligonukleotidpaar HsgD von 331 bp, Oligonukleotidpaar HsgE von 399 bp und Oligonukleotidpaar HsgF von 586 bp. Die als Kontrolle verwendeten Virus-DNA-Proben wurden ebenfalls von Frau Dr. Pring-Akerbloom zur Verfügung gestellt (vergl. Beispiel 5: Subgenus A: Ad18, Subgenus B: Ad7, Subgenus C: Ad1, Subgenus D: Ad17:, Subgenus E: Ad4:, Subgenus F: Ad41)

Folgender PCR-Ansatz wurde verwendet:
500 ng DNA (Gewebe, Zellkultur) bzw. 50 ng Virus-DNA
1.5 mM MgCl₂
je 0.5 µM Primer
5 µl 10xPCR-Puffer ohne MgCl₂ (Sigma)
200 µM dNTP
2.5 U Taq-Polymerase (Sigma)

Jeder Ansatz enthielt 50 µl Gesamtvolumen. Folgende Zyklen wurden durchgeführt:

| | | |
|---|---|---|
| 1× | 6 min | 95°C |
| 40× | 40 sec | 92°C |
| | 30 sec | 41°C |
| | 40 sec | 72 °C |
| 1 × | 5 min | 72°C |

Der gesamte Ansatz wurde in einem 1,5%igen Agarosegel aufgetragen. Mit den Oligonukleotidpaaren HsgA1/HsgA2, HsgB1/HsgB2, HsgC1/HsgC2 und HsgF1/HsgF2 wurde kein Fragment der jeweils erwarteten Länge aus den verschiedenen Lungenharmartom-DNA-Proben amplifiziert werden, wobei das entsprechende Fragment aus der viralen Kontroll-DNA amplifiziert wurde. Aus den DNA-Proben von 3 Lungenhamartomen wurde mit den Oligonucleotiden HsgD1/HsgD2 ein 331 bp Fragment amplifiziert. Aus 4 weiteren Lungenhamartom-DNA-Proben wurde mit dem Oligonucleotidpaar HsgF1/HsgF2 ein 399 bp langes Produkt amplifiziert.

Damit wurde eindeutig gezeigt, daß eine Infektion der Ursprungszelle mit Adenoviren der Gruppe D oder E zur Bildung von Lungenhamartomen führt.

### Beispiel 10

Um zu prüfen, ob Zellen von Lungenhamartomen in vitro permissiv sein können, wurden Hela-Zellen mit Zellen eines Lungenhamartoms kokultiviert. Hela-Zellen werden häufig für die Vermehrung von Adenoviren benutzt und zeigen nach Infektion cytopathologische Effekte.

Das verwendete Hamartomgewebe stammte von einem Tumor mit einer chromosomalen Translokation t(6;14)(p21;q24). Er wurde nach der Operation für 26 Stunden in Hank's Lösung bei Raumtemperatur gelagert und dann mit Schere und Skalpell in ca. 1 mm³ große Würfel zerkleinert. Nach Routinemethoden erfolgte dann enzymatisch die weitere Zerkleinerung mit Kollagenase (Kazmierczak et al., Oncogene, 12: 515 - 521). Die resultierende Zellsuspension wurde auf vier 25 cm² Zellkulturflaschen verteilt, die jeweils mit 5 ml Zellkulturmedium (Medium 199 mit 20 % fetalem Kälberserum unter Zusatz von Antibiotika) vorgefüllt waren. Nach dreitägiger Kultivierungsdauer bei 3°C, 5 % CO₂ hatte sich in den Flaschen ein konfluenter Monolayer gebildet. Die Zellen wurden daraufhin nach Standardverfahren mit Trypsin/EDTA-Lösung abgelöst und jeweils auf zwei neue Kulturflaschen verteilt. Nach zwei Stunden wurden zu den Kulturflaschen jeweils ca. 3x10⁵ Hela-Zellen in 1 ml Kulturmedium gegeben. Nach eintägiger Kulturauer enthielten die Flaschen je etwa 50 % von Heia-Zellen und fibroblastenartigen Hamartom-Zellen. Die Serumkonzentration im Medium wurde auf 10 % reduziert. Nach zwei weiteren Tagen zeigten die Hela-Zellen an mehreren Stellen Veränderungen der Zellmorphologie und begannen sich elipsenartige Zellen vom Boden der Kulturgefäße abzulösen. Nach vier Tagen waren nur noch einzelne Gruppen von Hela-Zellen nachzuweisen, die allerdings proliferierten.

Der aufgetretene cytopathologische Effekt kann auf für Adenoviren permissive Hamartom-zellen zurückgeführt werden, die Hela-Zellen infiziert haben.

### Beispiel 11

Unter der Annahme daß, eine Aktivierung von Genen der HMGI(Y)-Familie bzw. deren erhöhte Expression zu einem verstärkten Wachstum Adenovirus-transformierter Zellen führt, wurde zur Abklärung das folgende Experiment durchgeführt:
Eine Hamsterzellinie, die ein einzelnes Integrat des menschlichen Adenovirus im Genom aufweist, wurde als Empfänger für das Transfektionsexperiment benutzt. In den Zellen wurde mittels eines Expressionsvektors für das "Wildtyp"-HMGIC, wie in Beispiel 4 beschrieben, das HMGIC-Gen transient überexprimiert. Mit "leerem" Vektor transfizierte Zellen dienten als Negativkontrolle. In den Ansätzen wurden 12 Stunden, 24 Stunden und 36 Stunden nach Transfektion die Zellzahlen bestimmt. Während nach 12 Stunden noch keine Unterschiede zwischen den Kulturen deutlich wurden, wiesen die Kulturen mit Überexpression des HMGIC nach 24 und 36 Stunden gegenüber den Kontrollkulturen durchschnittlich um den Faktor 1,2 bzw. 1,47 erhöhte Zellzahlen auf. Diese Transfektionsexperimente zeigen damit den Wachstumsvorteil auf, der durch Überexpression von HMGIC in Adenovirus-transformierten Zellen vermittelt wird und bestätigt somit die obige Auffassung, daß adenoviral transformierte Zellen bei Aktivierung von Genen der HMGI(Y)-Familie ein verstärktes Wachstum zeigen.

### Beispiel 12

Zur Untersuchung von Endometrioseherden auf das Vorkommen von Adenovirus der Gruppe B, die auch bei Uterus-Leiomyomen nachgewiesen werden konnten, wurden sofort nach Operation Gewebeproben von makroskopisch identifizierten Endometrioseherden von 4 Patientinnen in flüssigen Stickstoff eingefroren. Aus den eingefrorenen Proben wurde dann nach üblichen Methoden DNA isoliert. Diese DNA wurde für die in Beispiel 5 beschriebene PCR-Analyse eingesetzt. In 2 der analysierten Proben fand sich eine Amplifikation des für Adenovieren der Gruppe B typischen 465bp großen Fragments. Aus den Ergebnissen einer Verdünnungsreihe, bei der Virus-Kontroll-DNA aus Myometrium, bei der keine Amplifikation nachgewiesen werden konnte, gemischt wurde, konnte gezeigt werden, daß mit der Methode nur mehr als durchschnittlich 2 Virusgenome pro Wirtszelle nachgewiesen werden können. Aus den Ergebenissen der hier dargestellen Analyse läßt sich also festellen, daß im Fall der zwei positiven Proben durchschnittlich mehr als 2 Virusgenome pro Zelle vorliegen müssen.

### Beispiel 13

Zur Untersuchung von Endometriumpolypen auf das Vorkommen von Adenovirus der Gruppe B, die auch bei Uterus-Leiomyomen nachgewiesen werden konnten, wurden sofort nach Operation Gewebeproben von makroskopisch identifizierten Endometriumpolypen von 3 Patientinnen in flüssigen Stickstoff eingefroren. Aus den eingefrorenen Proben wurde dann nach üblichen Methoden DNA isoliert. Diese DNA wurde für die in Beispiel 5 beschriebene PCR-Analyse eingesetzt. In einer der analysierten Proben fand sich eine Amplifikation des für Adenovieren der Gruppe B typischen 465bp großen Fragments. Aus den Ergebnissen einer Verdünnungsreihe, bei der Virus-Kontroll-DNA mit DNA aus Myometrium, bei der keine Amplifikation nachgewiesen werden konnte, gemischt wurde, konnte gezeigt werden, daß mit der Methode nur mehr als durchschnittlich 2 Virusgenome pro Wirtszelle nachgewiesen werden können. Aus den Ergebnissen der hier dargestellten Analyse läßt sich also feststellen, daß im Fall der positiven Probe durchschnittlich mehr als 2 Virusgenome pro Zelle vorliegen müssen.

## Patentansprüche

1. Verwendung eines Mittels, das ein antiviral wirksames Agens umfasst, zur Herstellung eines Medikamentes für die Prävention und/oder Therapie von Gewebeveränderungen wobei
- die Gewebeveränderung Gewebe mesenchymalen Ursprungs umfasst, die Gewebeveränderung eine Proliferation mindestens einer mesonchymalen Zelle ist, die mit einem Virus infiziert ist, wobei die Gewebeveränderung ausgewählt ist. aus der Gruppe, die Leiomyome, insbesondere Leiomyome des Uterus, Endometriumpolypen, Endometriose, Fibroadenome, insbesondere Fibroadenome der Mamma, Phylloides-Tumoren, insbesondere der Mamma, Harmatome, insbesondere der Mamma und der Lunge, Prostata-Adenome, Lipome, aggressive Angiomyxome, Enchondrome, pleomorphe Adenome, insbesondere der Kopfspeicheldrüsen, Kolon-Polypen, insbesondere Kolon-Adenome, Atherome und daraus entstandene Karzinome umfasst; wobei die Nukleinsäure des Virus mindestens eine Bindungsstelle für ein Genprodukt von Genen der HMGI(Y)-Familie umfaßt oder das Virus für ein solches Genprodukt codiert, wobei das Virus ausgewählt ist aus der Gruppe, die Adenoviren, Herpesviren und Papova-viren enthält und das Agens gegen das Virus genichtet ist, wobei das Agens aus der Gruppe ausgewählt ist, die Impfstoffe gegen das Virus, Antikörper gegen das Virus, Adenosinarabinosid, Bromvinylundinarabinosid, 9.(1,3-Dihydroxy-2-Propoxy) Methylguanin, Trinatriumsalz der Phosphoroameisensäure, Interferon α, Interferon β und Interferon γ enthält.

2. Verwendung nach Anspruch 1, **dadurch gekennzeichnet, dass** das Mittel wirksam ist gegen ein Virus, dessen Nukleinsäure für ein Genprodukt codiert, wobei dieses Genprodukt mindestens mit einem Genprodukt von Genen der HMGI(Y)-Familie oder deren Derivaten wechselwirkt.

3. Verwendung nach Anspruch 1, **dadurch gekennzeichnet, dass** die Bindungsstelle auf der Nukleinsäure des Virus das Struktur- und Sequenzmerkmal einer ersten AT-reichen Sequenz umfasst.

4. Verwendung nach Anspruch 3, **dadurch gekennzeichnet, dass** die Bindungsstelle auf der Nukleinsäure neben der ersten Sequenz noch die Struktur- und Sequenzmerkmale umfasst, dass
- eine zweite AT-reiche Sequenz vorhanden ist, und
- die erste und zweite Sequenz in einer räumlichen Distanz zueinander angeordnet sind.

5. Verwendung nach Anspruch 4, **dadurch gekennzeichnet, dass** die räumliche Distanz so ausgewählt ist, dass die erste Sequenz und die zweite Sequenz relativ zueinander in einer Ebene auf der Nukleinsäure angeordnet sind.

6. Verwendung nach einem der Ansprüche 1 bis 5, **dadurch gekennzeichnet, dass** die Gene der HMGI(Y)-Familie MAG-Gene, HMGIC, HMGIY und aberrante Transkripte von Genen der HMGI(Y)-Familie und Derivate davon umfassen.

7. Verwendung nach einem der Ansprüche 1 bis 6, **dadurch gekennzeichnet, dass** das mindestens eine Zelle der das Gewebe aufbauenden Gewebeveränderung infizierende Virus ein solches nach einem der vorangehenden Ansprüche ist.

8. Verwendung nach einem der Ansprüche 1 bis 7, **dadurch gekennzeichnet, dass** das Mittel wirksam ist gegen Adenoviren und/oder Herpesviren.

9. Verwendung nach einem der Ansprüche 1 bis 8, **dadurch gekennzeichnet, dass** das Gewebe mesenchymalen Ursprungs zumindest teilweise mit einem Virus aus der Gruppe der Adeno- und/oder Herpesvirus infiziert ist.

10. Verwendung nach einem der Ansprüche 1 bis 9, **dadurch gekennzeichnet, dass** die Gewebeveränderung eine Proliferation mindestens einer mesenchymalen Zelle, die mit einem Virus nach den vorangehenden Ansprüchen infiziert ist, umfasst.

11. Verwendung nach Anspruch 10, **dadurch gekennzeichnet, dass** die Proliferation eine klonale Proliferation ist.

12. Verwendung nach einem der Ansprüche 1 bis 11, **dadurch gekennzeichnet, dass** die Gewebeveränderung eine epitheliale Komponente umfasst.

13. Verwendung nach Anspruch 12, **dadurch gekennzeichnet, dass** die epitheliale Komponente mindestens eine Zelle aufweist, die mit einem Virus nach einem der vorangehenden Ansprüche infiziert ist.

14. Verwendung nach einem der Ansprüche 1 bis 13, **dadurch gekennzeichnet, dass** die mit einem Virus infizierte Zelle eine chromosomale Veränderung aufweist.

15. Verwendung nach Anspruch 14, **dadurch gekennzeichnet, dass** die chromosomale Veränderung mindestens ein HMGI(Y)-Gen der infizierten Zelle umfasst.

16. Verwendung nach Anspruch 15, **dadurch gekennzeichnet, dass** das HMHI(Y)-Gen aus der Gruppe ausgewählt ist, die MAG-Gene, HMGIC, HMGIY und aberrante Transkripte von Genen der HMGI(Y)-Familie und Derivate umfasst.

17. Verwendung nach Anspruch 1, **dadurch gekennzeichnet, dass** die entstandenen Karzinome aus der Gruppe ausgewählt sind, die Kolon-Karzinome und Prostata-Karzinome umfasst.

18. Verwendung nach Anspruch 1, **dadurch gekennzeichnet, dass** der Impfstoff ein Partikel eines Virus nach einem der vorangehenden Ansprüche oder einen Teil davon umfasst.

19. Verwendung nach Anspruch 1, **dadurch gekennzeichnet, dass** der Antikörper ausgewählt ist aus der Gruppe, die monoklonale Antikörper, polyklonale Antikörper, polyvalente Antikörper, Antikörperfragmente und Derivate davon umfasst.

20. In vitro Verwendung eines Diagnoseverfahrens, wobei eine Körperflüssigkeit von einem eine Gewebeveränderung möglicherweise aufweisenden Patienten auf das Vorhandensein von Antikörpern gegen oder Antigene von Viren, wie beschrieben in einem der vorangehenden Ansprüche, untersucht wird, zur Diagnose einer Gewebeveränderung, wobei die Gewebeveränderung eine solche nach einem der vorangehenden Ansprüche umfasst.

21. In vitro Verwendung eines Diagnoseverfahrens, wobei eine Gewebeprobe mit einem Mittel umgesetzt wird, das aus der Gruppe ausgewählt ist, die Antikörper, die mit Viren, wie beschrieben in einem der vorangehenden Ansprüche, oder Teilen davon reagieren; Antigene, die von Viren, wie beschrieben in einem der vorangehenden Ansprüche, oder Teilen davon stammen; und Nukleinsäure, die mit Nukleinsäure von Viren wie beschrieben in einem der vorangehenden Ansprüche, wechselwirkt, umfasst, zur Diagnose einer Gewebeveränderung, **dadurch gekennzeichnet, dass**
a) die Gewebeveränderung eine solche nach einem der vorangehenden Ansprüche umfasst, und
b) im Falle der Anwesenheit von Viren wie beschrieben in einem der vorangehenden Ansprüche, sich ein Komplex aus dem Mittel und dem Virus bildet, und
c) der Komplex nachgewiesen wird.

## Claims

1. Use of a preparation which comprises an antiviral agent for the manufacture of a medicament for preventing and/or treating tissue changes, whereby the tissue change comprises tissue of mesenchymal origin, the tissue change is a proliferation of at least one mesenchymal cell which is infected with a virus, whereby the tissue change is selected from the group comprising leiomyomas, in particular leiomyomas of the uterus, endometrial polyps, endometriosis, fibroadenomas, in particular fibroadenomas of the mamma, phylloides tumours, in particular of the mamma, hamartomas, in particular of the mamma and the lung, prostate adenomas, lipomas, aggressive angiomyxomas, enchondromas, pleomorphic adenomas, especially of the salivary glands of the head, colon polyps, in particular colon adenomas, atheromas and carcinomas that have developed therefrom, whereby the nucleic acid of the virus comprises at least one binding site for a gene product of genes of the HMGI(Y) family or the virus codes for such a gene product, whereby the virus is selected from the group comprising adenoviruses, herpes viruses and papova viruses, and the agent is selected from the group comprising vaccines against the virus, antibodies against the virus, adenosine arabinoside, bromovinyluridine arabinoside, 9-(1,3-dihydroxy-2-propoxy)methyl guanine, trisodium salt of phosphoroformic acid, interferon α, interferon β and interferon γ.

2. Use according to claim 1, **characterized in that** the preparation is effective against a virus the nucleic acid of which codes for a gene product, whereby the gene product interacts with at least one gene product of genes of the HMGI(Y) family or derivatives thereof.

3. Use according to claim 1, **characterized in that** the binding site on the nucleic acid of the virus has the structural and sequence feature of a first AT-rich sequence.

4. Use according to claim 3, **characterized in that** the binding site on the nucleic acid, in addition to the first sequence, also has the following structural and sequence features:
- a second AT-rich sequence is present, and
- the first and second sequence are arranged at a spatial distance from one another.

5. Use according to claim 4, **characterized in that** the spatial distance is selected such that the first sequence and the second sequence are arranged on the nucleic acid relative to one another in one plane.

6. Use according to any of claims 1 to 5, **characterized in that** the genes of the HMGI(Y) family comprise MAG genes, HMGIC, HMGIY, and aberrant transcripts of genes of the HMGI(Y) family and derivatives thereof.

7. Use according to any of claims 1 to 6, **characterized in that** the virus infecting at least one cell of the tissue forming the tissue change is one as defined in any of the preceding claims.

8. Use according to any of claims 1 to 7, **characterized in that** the preparation is effective against adenoviruses and/or herpes viruses.

9. Use according to any of claims 1 to 8, **characterized in that** the tissue of mesenchymal origin is at least partially infected with a virus from the group of adenoviruses and/or herpes viruses.

10. Use according to any of claims 1 to 9, **characterized in that** the tissue change comprises a proliferation of at least one mesenchymal cell which is infected with a virus as defined in any of the preceding claims.

11. Use according to claim 10, **characterized in that** the proliferation is a clonal proliferation.

12. Use according to any of claims 1 to 11, **characterized in that** the tissue change comprises an epithelial component.

13. Use according to claim 12, **characterized in that** the epithelial component comprises at least one cell which is infected with a virus as defined in any of the preceding claims.

14. Use according to any of claims 1 to 13, **characterized in that** the cell infected with the virus has a chromosomal change.

15. Use according to claim 14, **characterized in that** the chromosomal change comprises at least one HMGI(Y) gene of the infected cell.

16. Use according to claim 15, **characterized in that** the HMGI(Y) gene is selected from the group comprising MAG genes, HMGIC, HMGIY, and aberrant transcripts of genes of the HMGI(Y) family and derivatives thereof.

17. Use as claimed in claim 1, **characterized in that** the carcinomas that have developed, are selected from the group comprising colon carcinomas and prostate carcinomas.

18. Use according to claim 1, **characterized in that** the vaccine contains a particle of a virus as defined in any of the preceding claims or a part thereof.

19. Use according to claim 1, **characterized in that** the antibody is selected from the group comprising monoclonal antibodies, polyclonal antibodies, polyvalent antibodies, antibody fragments and derivatives thereof.

20. In vitro use of a diagnostic method, whereby a body fluid from a patient who may have a tissue change, is examined for the presence of antibodies against viruses or antigens of viruses as described in any of the preceding claims, for the diagnosis of a tissue change, whereby the tissue change comprises a tissue change as defined in any of the preceding claims.

21. In vitro use of a diagnostic method, whereby a tissue sample is reacted with a means which is selected from the group comprising antibodies which react with viruses as described in any of the preceding claims or parts thereof, antigens that are derived from viruses as described in any of the preceding claims or parts thereof, and a nucleic acid which interacts with a nucleic acid of any of the viruses as described in any of the preceding claims, for the diagnosis of a tissue change, **characterized in that**
a) the tissue change comprises a tissue change as defined in any of the preceding claims, and
b) if viruses as described in any of the preceding claims are present, a complex of the means and the virus is formed and
c) said complex is detected.

## Revendications

1. Utilisation d'un produit contenant un agent actif antiviral pour préparer un médicament destiné à la prévention et/ou au traitement de modifications tissulaires, dans laquelle
- la modification tissulaire comprend un tissu d'origine mésenchymateuse, la modification tissulaire est une prolifération d'au moins une cellule mésenchymateuse qui est infectée par un virus, ladite modification tissulaire étant choisie dans le groupe comprenant des liomyomes, notamment des liomyomes de l'utérus, des polypes de l'endomètre, l'endométriose, des fibroadénomes, notamment des fibroadénomes du sein, des tumeurs phyllodes, notamment du sein, des hamartomes, notamment du sein et du poumon, des adénomes de la prostate, des lipomes, des angiomyxomes agressifs, des enchondromes, des adénomes pléomorphes, notamment des glandes salivaires, des polypes du côlon, notamment des adénomes du côlon, des athéromes et les carcinomes qui en résultent, l'acide nucléique du virus comprenant au moins un site de liaison pour un produit génique de gènes de la famille HMGI(Y) ou le virus codant pour un tel produit génique, ledit virus étant choisi dans le groupe comprenant des adénovirus, des herpèsvirus et des papovavirus, et l'agent actif est dirigé contre le virus, ledit agent actif étant choisi dans le groupe comprenant des vaccins contre le virus, des anticorps dirigés contre le virus, l'adénosine arabinoside, la bromovinyluridine arabinoside, la 9-(1,3-dihydroxy-2-propoxy)méthylguanine, le sel trisodique de l'acide phosphonoformique, l'interféron α, l'interféron β et l'interféron γ.

2. Utilisation selon la revendication 1, **caractérisée en ce que** le produit est efficace contre un virus dont l'acide nucléique code pour un produit génique, ledit produit génique présentant une interaction avec au moins un produit génique de gènes de la famille HMGI(Y) ou de leurs dérivés.

3. Utilisation selon la revendication 1, **caractérisée en ce que** le site de liaison sur l'acide nucléique du virus comprend la caractéristique de structure et de séquence d'une première séquence riche en AT.

4. Utilisation selon la revendication 3, **caractérisée en ce que** le site de liaison sur l'acide nucléique, outre la première séquence, comprend en plus les caractéristiques de structure et de séquence selon lesquelles
- une deuxième séquence riche en AT est présente, et
- la première et la deuxième séquences sont disposées à une distance spatiale l'une de l'autre.

5. Utilisation selon la revendication 4, **caractérisée en ce que** la distance spatiale est choisie telle que la première séquence et la deuxième séquence sont disposées l'une par rapport à l'autre dans un plan sur l'acide nucléique.

6. Utilisation selon l'une des revendications 1 à 5, **caractérisée en ce que** les gènes de la famille HMGI(Y) comprennent des gènes MAG, HMGIC, HMGIY et des produits de transcription aberrants de gènes de la famille HMGI(Y) ainsi que des dérivés de ceux-ci.

7. Utilisation selon l'une des revendications 1 à 6, **caractérisée en ce que** le virus infectant au moins une cellule de la modification tissulaire constituant le tissu est un virus selon l'une des revendications précédentes.

8. Utilisation selon l'une des revendications 1 à 7, **caractérisée en ce que** le produit est efficace contre des adénovirus et/ou des herpèsvirus.

9. Utilisation selon l'une des revendications 1 à 8, **caractérisée en ce que** le tissu d'origine mésenchymateuse est infecté au moins en partie par un virus du groupe des adénovirus et/ou herpèsvirus.

10. Utilisation selon l'une des revendications 1 à 9, **caractérisée en ce que** la modification tissulaire comprend une prolifération d'au moins une cellule mésenchymateuse qui est infectée par un virus selon les revendications précédentes.

11. Utilisation selon la revendication 10, **caractérisée en ce que** la prolifération est une prolifération clonale.

12. Utilisation selon l'une des revendications 1 à 11, **caractérisée en ce que** la modification tissulaire comprend une composante épithéliale.

13. Utilisation selon la revendication 12, **caractérisée en ce que** la composante épithéliale présente au moins une cellule qui est infectée par un virus selon l'une des revendications précédentes.

14. Utilisation selon l'une des revendications 1 à 13, **caractérisée en ce que** la cellule infectée par un virus présente une modification chromosomique.

15. Utilisation selon la revendication 14, **caractérisée en ce que** la modification chromosomique comprend au moins un gène HMGI(Y) de la cellule infectée.

16. Utilisation selon la revendication 15, **caractérisée en ce que** le gène HMHI(Y) est choisi dans le groupe comprenant des gènes MAG, HMGIC, HMGIY et des produits de transcription aberrants et des dérivés de gènes de la famille HMGI(Y)

17. Utilisation selon la revendication 1, **caractérisée en ce que** les carcinomes résultants sont choisis dans le groupe comprenant des carcinomes du côlon et des carcinomes de la prostate.

18. Utilisation selon la revendication 1, **caractérisée en ce que** le vaccin comprend une particule d'un virus ou d'une partie de virus selon l'une des revendications précédentes.

19. Utilisation selon la revendication 1, **caractérisée en ce que** l'anticorps est choisi dans le groupe comprenant des anticorps monoclonaux, des anticorps polyclonaux, des anticorps polyvalents, des fragments d'anticorps et des dérivés de ceux-ci.

20. Utilisation *in vitro* d'un procédé de diagnostic, dans lequel un fluide corporel d'un patient susceptible de présenter une modification tissulaire est analysé afin de rechercher la présence d'anticorps dirigés contre, ou d'antigènes de, virus tels que décrits dans l'une des revendications précédentes, pour le diagnostic d'une modification tissulaire, ladite modification tissulaire comprenant une modification selon l'une des revendications précédentes.

21. Utilisation *in vitro* d'un procédé de diagnostic, dans lequel on fait réagir un échantillon de tissu avec un produit choisi dans le groupe comprenant des anticorps qui réagissent avec des virus ou des parties de virus tels que décrits dans l'une des revendications précédentes ; des antigènes issus de virus ou de parties de virus tels que décrits dans l'une des revendications précédentes ; et un acide nucléique qui présente une interaction avec un acide nucléique de virus tels que décrits dans l'une des revendications précédentes, pour le diagnostic d'une modification tissulaire, **caractérisée en ce que**
a) la modification tissulaire comprend une modification selon l'une des revendications précédentes, et
b) en cas d'absence de virus tels que décrits dans l'une des revendications précédentes, il se forme un complexe entre le produit et le virus, et
c) le complexe est déterminé.
